# EUROPEAN PATENT SPECIFICATION

(11) **EP 2 081 100 B1**
(45) Date of publication and mention of the grant of the patent: **08.08.2012**
(21) Application number: 07831830.0
(22) Date of filing: 14.11.2007
(51) Int. Cl.: A61B 5/0484, G06F 3/01

(54) **ADJUSTING DEVICE FOR BRAIN WAVE IDENTIFICATION METHOD, ADJUSTING METHOD AND COMPUTER PROGRAM**
EINSTELLVORRICHTUNG FÜR EIN VERFAHREN ZUR ERFASSUNG VON GEHIRNWELLEN SOWIE EINSTELLVERFAHREN UND COMPUTERPROGRAMM DAFÜR
DISPOSITIF D'AJUSTEMENT POUR UN PROCÉDÉ D'IDENTIFICATION D'ONDES CÉRÉBRALES, PROCÉDÉ D'AJUSTEMENT ET PROGRAMME INFORMATIQUE

(30) Priority: 15.11.2006 JP 2006309385
(43) Date of publication of application: 22.07.2009
(73) Proprietor: Panasonic Corporation, Kadoma-shi Osaka 571-8501 (JP)
(72) Inventor: MORIKAWA, Koji c/o Panasonic Corporation IPROC, Osaka-shi, Osaka 540-6207 (JP); ADACHI, Shinobu c/o Panasonic Corporation IPROC, Osaka-shi, Osaka 540-6207 (JP)
(74) Representative: Grünecker, Kinkeldey, Stockmair & Schwanhäusser
(86) International application number: PCT/JP2007/072100
(87) International publication number: WO 2008/059878

(56) References cited:
- WO-A1-2006/051709
- JP-A- 2006 023 835
- US-A1- 2005 085 744
- EBRAHIMI T ET AL: "Brain-Computer Interface in Multimedia Communication" IEEE SIGNAL PROCESSING MAGAZINE, IEEE SERVICE CENTER, PISCATAWAY, NJ, US, vol. 20, no. 1, 1 January 2003 (2003-01-01), pages 14-24, XP011093777 ISSN: 1053-5888
- WOLPAW J R ET AL: "Brain-computer interfaces for communication and control" CLINICAL NEUROPHYSIOLOGY ELSEVIER IRELAND, vol. 113, no. 6, June 2002 (2002-06), pages 767-791, XP002551582 ISSN: 1388-2457

## Description

### TECHNICAL FIELD

The present invention relates to an interface (electroencephalogram interface) system which makes it possible to manipulate a device by utilizing electroencephalograms. More specifically, the present invention relates to an electroencephalogram interface system which detects an intent to manipulate a device or acquire information by detecting a psychological state, emotional state, cognitive state or the like of a user through utilization of the user's electroencephalograms, such that the electroencephalogram interface system has a function of performing a calibration for enabling precise analysis of electroencephalograms.

### BACKGROUND ART

To date, various types of devices such as information devices, e.g., televisions, mobile phones, and PDAs (Personal Digital Assistants), have been proposed. Users lead their everyday lives amongst such devices, and enjoy desired information and services through manipulation of these devices. The number of devices has always been on the increase; the information that cannot be obtained without using the devices is increasing; and also for other reasons, the importance of improving the manipulability of interfaces for device manipulation has been increasing year after year.

For example, as for the aforementioned information devices, device manipulation has been realized by selecting a desired manipulation alternative while watching a screen of a device. As a means of manipulation inputting, methods such as pressing a button, moving a cursor and making a decision, or manipulating a mouse while watching the screen, have been used, for example. However, in the case where both hands are unavailable due to any work other than device manipulations, e.g., household chores, rearing of children, or driving of an automobile, it may be difficult to make an input by utilizing the manipulation input means, thus rendering the device manipulation impossible. This has promoted the users' need to manipulate an information device even in a situation where both hands are full.

In answer to such needs, input means utilizing biological signals from a user has been developed.
For example, US2005085744 describes a system for developing a brain-computer interface (BCI), especially for use in rehabilitation, includes an audio-visual interface device for applying to a subject being examined stimuli eliciting event-related potentials and inducing brain reactions in said subject being examined. The system further includes an acquisition device for acquiring brain reaction signals (such as EEG traces) of the subject being examined synchronized with the stimuli and at least one processing device for processing the signals acquired via said acquisition device, The interface device, the acquisition device and the processing device comprise an integrated system. Preferably, the system uses a p 300 signal as the event-related potential.

In another example, Non-Patent Document 1 discloses an electroencephalogram interface technique that utilizes an event-related potential of electroencephalograms for distinguishing an alternative which a user wishes to select. To specifically describe the technique described in Non-Patent Document 1, alternatives are randomly highlighted, and the waveform of an event-related potential which appears about 300 milliseconds after a point in time that an alternative was highlighted is utilized to enable distinction of the alternative which the user wishes to select. According to this technique, even in a situation where both hands are full, or even in a situation where the user is unable to move his or her limbs due to an illness or the like, the user can select an alternative which they wish to select, whereby an interface for device manipulations, etc., that satisfies the aforementioned needs is realized.

As used herein, an "event-related potential" refers to a transient potential fluctuation in the brain which occurs in temporal relationship with an external or internal event. An electroencephalogram interface utilizes this event-related potential as measured from a starting point which is the point in time when an external event occurs. For example, selection of a menu alternative is supposed to be possible by utilizing a component of an event-related potential called "P300" which occurs in response to a visual stimulation or the like. "P300" is a positive component of an event-related potential which appears near about 300 milliseconds from the starting point.

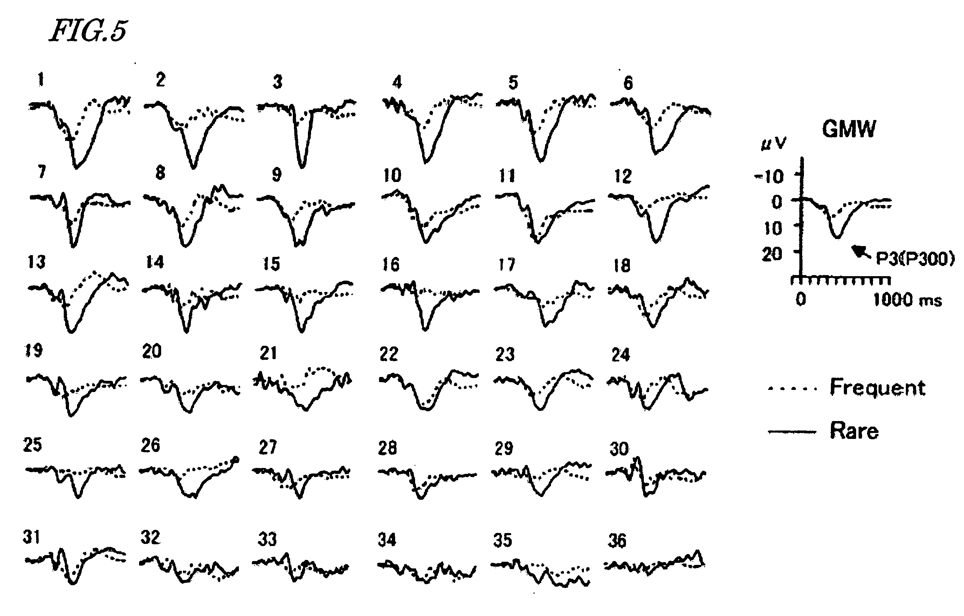

Since there are large individual differences in the manner in which the aforementioned electroencephalogram waveforms may appear, it is necessary to realize a highly accurate distinction that supports such individual differences, in order to utilize an event-related potential as the input means for an interface. Examples of individual differences are shown at page 32 of Non-Patent Document 1. Moreover, FIG. **5** shows examples of individual differences in electroencephalograms when the same problem is presented to 36 examinees. In the graph of each examinee, electroencephalograms for 2 kinds of situations are presented, as shown by a solid line and a broken line. As is clear from FIG. **5****,** it can be said that it is difficult to accurately perform distinction for every user by relying on a single criterion, because there is great variation in the waveform and amplitude at the peak position, due to individual differences.

In order to correct for individual differences and accurately measure electroencephalograms (biological signals), it is necessary to calibrate the measurement equipment and make adjustments so that the data which is measured with the measurement equipment accurately represents biological information of a user. For example, in order to accurately measure a line of sight of a user, Patent Document 1 discloses a technique of calibrating measurement equipment at the time when the measurement equipment is worn, i.e., before a measurement of the line of sight, thus establishing matching in a coordinate system between the measurement equipment and the user's line of sight.

On the other hand, there have also been devised appropriate distinction methods which take into account the individual differences appearing in a component of the event-related potential. For example, Patent Document 2 discloses a technique of obtaining an improved distinction ratio by changing the distinction method for each user. In this technique, instead of performing distinction for all users with a single criterion, a template for each individual is prepared in advance from an arithmetic mean waveform of an event-related potential for a situation to be examined, and a component of the event-related potential is distinguished by using this template. See Non-Patent Document 2 for details of individual differences in event-related potentials, for example.

Conventional electroencephalogram interfaces have been taken farther as a study directed to people who are incapable of free body movements, with an aim to realize an interface for device manipulation by utilizing the fact that electroencephalograms can still be freely expressed despite incapability of free body movements. Non-Patent Document 1, supra, shows that use of a P300 component of the event-related potential makes possible a menu selection, text input, and the like. This has made it possible to express a request for something to drink or a treatment, onto a screen which is prepared near the bed in a state which is ready to be used at all times, with a menu being displayed thereon.

Also in the case of applying the aforementioned electroencephalogram interface to the manipulation of a device for daily use by users at large, it is indispensable to adjust the system for each user in order to permit accurate distinction of the event-related potential of each user sharing large individual differences. According to the concept of traditional calibration, a method would be conceivable in which a task of virtually manipulating an electroencephalogram interface is performed before using the electroencephalogram interface, and which records a corresponding waveform for use as information in making a distinction when the electroencephalogram interface is actually used.
[Patent Document 1] Japanese Laid-Open Patent Publication No. 2005-312605
[Patent Document 2] Pamphlet of International Laid-Open No. 06/051709 (paragraph [0068])
[Non-Patent Document 1] (Emanuel Donchin) and two others, "The Mental Prosthesis: Assessing the Speed of a P300-Based Brain-Computer Interface", TRANSACTIONS ON REHABILITATION ENGINEERING, Vol.8, No.2, June 2000
[Non-Patent Document 2] Hiroshi NITTONO, "Event-related Potential Guidebook for Psychological Research", KITAOJI SHOBO, issued on September 20, 2005, p. 32

### DISCLOSURE OF INVENTION

### PROBLEMS TO BE SOLVED BY THE INVENTION

However, when the electroencephalogram interface is applied to the manipulation of a device for daily use, it is a burden and trouble to the user that a ceremonious calibration must be executed in order for the expected function to be exhibited. It would be absurd if an electroencephalogram interface intended to reduce the burden of a user were to conversely become a burden to the user. Therefore, when an electroencephalogram interface is applied to daily purposes, a user should be able to casually use it at any time, and the electroencephalogram interface should accurately operate so as to exhibit its expected function.

However, when an electroencephalogram interface is applied to daily purposes, there exist aspects which would not be deemed problematic in special situations such as research or medical applications. For example, one aspect is that casual manipulations such as changing of a channel or changing of sound volume level would not be frequently made, and there is no knowing when they would be made.

Another aspect is that information other than a menu for the electroencephalogram interface is usually being indicated on the display, thus exerting a large influence on the event-related potential, together with its irregular occurrences. For example, in any period of time while the electroencephalogram interface is not activated, other information (television programs, movies, etc.) is being displayed. On the other hand, in the case of research or medical applications, it would be possible to perform a calibration by spending a sufficient time before using the interface and thus acquire electroencephalograms based on a sufficient ability for correction, in order to enhance the distinction accuracy of electroencephalograms.

Therefore, for an electroencephalogram interface, not being used all the time and yet having to accurately operate in a rare manipulation of a device is a vary harsh condition. A method is needed which precisely adjusts the electroencephalogram distinction method for each user.

One objective of the present invention is to, in a system having an interface which utilizes electroencephalograms, free a user from the burden of calibration for enabling accurate measurement of electroencephalograms, and yet maintain a high determination accuracy for electroencephalograms.

### MEANS FOR SOLVING THE PROBLEMS

The above problems and disadvantages of the prior art are solved by the present invention as claimed in the independent claims.

Advantageous and preferred embodiments of the invention are defined by the dependent claims.

An adjustment apparatus according to an example is used for adjusting a distinction method in an electroencephalogram interface section in an electroencephalogram interface system, the electroencephalogram interface system including: an output section for, based on data of a content, presenting the content to a user; a biological signal measurement section for acquiring an electroencephalogram signal from the user; and the electroencephalogram interface section for distinguishing a request of the user based on the electroencephalogram signal and identifying a function which is in accordance with the request. The adjustment apparatus comprises: an analysis section for measuring and analyzing a physical quantity corresponding to a visual and/or auditory stimulation given to the user, the physical quantity being measured and analyzed as a characteristic quantity of the stimulation, and for detecting a change in the characteristic quantity of the stimulation that affects an event-related potential contained in the electroencephalogram signal, thus detecting a change in the stimulation; a storage section for storing a waveform of the event-related potential during a predetermined period at least spanning after a starting point which is a point in time when the change in the stimulation is detected; a user characteristic extraction section for extracting a characteristic quantity of the user based on the stored waveform of the event-related potential; and a distinction method adjustment section for, based on the extracted characteristic quantity of the user, adjusting in the electroencephalogram interface section the distinction method for a request based on the electroencephalogram signal.

When the stimulation is video and/or audio of the content presented to the user, the analysis section may analyze the data of the content to detect a change in the characteristic quantity of the stimulation that affects the event-related potential contained in the electroencephalogram signal.

Based on moving picture data, the output section may display respectively a plurality of pictures which are switched one after another at a predetermined frequency, thus presenting a moving picture content; and as a change in the characteristic quantity of the content, the analysis section may detect a change in an image characteristic quantity of the consecutive plurality of images.

As a change in the characteristic quantity of the content, the analysis section may detect a change in at least one of luminance and hue.

The output section may present an audio content based on audio data; and as a change in the characteristic quantity of the content, the analysis section may detect a change in an output level of the audio.

The output section may present a moving picture content by displaying respectively a plurality of pictures which are switched one after another at a predetermined frequency, and also present an audio content; and as a change in the characteristic quantity of the content, the analysis section may detect a synchronized occurrence of a change in an image characteristic quantity of the consecutive plurality of images and a change in an output level of the audio.

The electroencephalogram interface section may distinguish the request of the user based on a threshold retained in advance and an amplitude of the event-related potential in the electroencephalogram signal; the user characteristic extraction section may extract an amplitude of the stored event-related potential as the characteristic quantity of the user; and based on the extracted characteristic quantity of the user, the distinction method adjustment section may adjust the threshold retained by the electroencephalogram interface section.

The electroencephalogram interface section may distinguish the request of the user based on a correlation between at least one waveform template retained in advance and the waveform of the event-related potential in the electroencephalogram signal; the user characteristic extraction section may extract the stored waveform of the event-related potential as the characteristic quantity of the user; and based on the extracted characteristic quantity of the user, the distinction method adjustment section may adjust the at least one waveform template retained by the electroencephalogram interface section.

Based on the extracted characteristic quantity of the user, the distinction method adjustment section may change a value of the at least one waveform template and set the at least one waveform template to the electroencephalogram interface section; and the electroencephalogram interface section may distinguish the request of the user based on the at least one waveform template having been set.

The electroencephalogram interface section may retain a plurality of waveform templates; and the distinction method adjustment section may identify one of the plurality of waveform templates based on the extracted characteristic quantity of the user, and instruct the electroencephalogram interface section to set the one waveform template as a template for distinguishing the request of the user.

The electroencephalogram interface section may distinguish the request of the user based on a correlation between a waveform template retained in advance and the waveform of the event-related potential in the electroencephalogram signal; the user characteristic extraction section may extract the stored waveform of the event-related potential as the characteristic quantity of the user; and the distinction method adjustment section may instruct one of the electroencephalogram interface section and the biological signal measurement section to adjust an amplitude of the electroencephalogram signal of the user based on the extracted characteristic quantity of the user.

The user characteristic extraction section may output a signal when an amplitude of the waveform the event-related potential stored in the storage section is smaller than a predetermined threshold; and based on the signal from the user characteristic extraction section, the distinction method adjustment section instructs the electroencephalogram interface section to output an alarm concerning acquisition of the electroencephalogram signal by the biological signal measurement section.

The stimulation may be light and/or sound in an environment within which the user exists; and the analysis section may detect light and/or sound in the environment, and detect a change in the characteristic quantity of light and/or sound in the environment that affects the event-related potential contained in the electroencephalogram signal.

A method according to an example is used for adjusting a distinction method in an electroencephalogram interface system, the electroencephalogram interface system including: an output section for, based on data of a content, presenting the content to a user; a biological signal measurement section for acquiring an electroencephalogram signal from the user; and an electroencephalogram interface section for distinguishing a request of the user based on the electroencephalogram signal and identifying a function which is in accordance with the request. The method comprises: a step of measuring a physical quantity corresponding to a visual and/or auditory stimulation given to the user, the physical quantity being measured as a characteristic quantity of the stimulation; a step of detecting a change in the characteristic quantity of the stimulation that affects an event-related potential contained in the electroencephalogram signal, thus detecting a change in the stimulation; a step of storing a waveform of the event-related potential during a predetermined period at least spanning after a starting point which is a point in time when the change in the stimulation is detected; a step of extracting a characteristic quantity of the user based on the stored waveform of the event-related potential; and a step of, based on the extracted characteristic quantity of the user, adjusting in the electroencephalogram interface section the distinction method for a request based on the electroencephalogram signal.

A computer program according to an example is executed by a computer implemented in an electroencephalogram distinction method adjustment apparatus used for adjusting a distinction method in an electroencephalogram interface section in an electroencephalogram interface system, the electroencephalogram interface system including: an output section for, based on data of a content, presenting the content to a user; a biological signal measurement section for acquiring an electroencephalogram signal from the user; and the electroencephalogram interface section for distinguishing a request of the user based on the electroencephalogram signal and identifying a function which is in accordance with the request. The computer program causes the computer to execute: a step of measuring a physical quantity corresponding to a visual and/or auditory stimulation given to the user, the physical quantity being measured as a characteristic quantity of the stimulation; a step of detecting a change in the characteristic quantity of the stimulation that affects an event-related potential contained in the electroencephalogram signal, thus detecting a change in the stimulation; a step of storing a waveform of the event-related potential during a predetermined period at least spanning after a starting point which is a point in time when the change in the stimulation is detected; a step of extracting a characteristic quantity of the user based on the stored waveform of the event-related potential; and a step of, based on the extracted characteristic quantity of the user, adjusting in the electroencephalogram interface section the distinction method for a request based on the electroencephalogram signal.

### EFFECTS OF THE INVENTION

According to the present invention, while a user is receiving external stimulations such as light and/or sound from a content which the user is viewing or from ambient light or environmental sound in an environment within which the user is situated, information concerning individual differences which are necessary for calibrating the electroencephalogram interface system is acquired. When the electroencephalogram interface is in use, a calibration is executed within the system by using the collected data.

Since the user is not forced to participate in a calibration, and no explicit calibration is performed, the burden and trouble to the user associated with calibration are greatly reduced. As a result, without any waiting time for a calibration, the user can immediately start viewing a content. On the other hand, since the calibration is performed with respect to each user, electroencephalograms can be accurately determined, whereby an electroencephalogram interface with an improved manipulability can be provided.

### BRIEF DESCRIPTION OF DRAWINGS

[FIG. **1**] A diagram showing a construction and an environment of use for an electroencephalogram interface system **1** as envisaged by the inventors.
[FIG. **2**] A diagram showing a functional block construction of the electroencephalogram interface system **1** according to Embodiment 1.
[FIG. **3**] A flowchart showing a procedure of processing by an electroencephalogram interface section **13.**
[FIG. **4**] **(a)** to **(d)** are diagrams showing an example where TV is manipulated in the electroencephalogram interface system **1** for a user **10** to watch a program of a genre that the user wishes to view.
[FIG. **5**] A diagram showing examples of individual differences in electroencephalogram signals.
[FIG. **6**] **(a)** is a diagram showing in chronological order a screen usage when performing a conventional calibration; and **(b)** is a diagram showing in chronological order a screen usage when performing a calibration according to Embodiment 1.
[FIG. **7**] A flowchart showing a procedure of processing by the electroencephalogram interface system **1** according to Embodiment 1.
[FIG. **8**] A flowchart showing the procedure of a process of analyzing a video content which is a moving picture.
[FIG. **9**] A flowchart showing the procedure of an extraction process for a characteristic of a user based on the waveform of an event-related potential.
[FIG. **10**] **(a)** to **(d)** are diagrams showing exemplary waveforms associated with a process of extracting a characteristic of a user.
[FIG. **11**] A diagram showing a template before adjustment and a template after adjustment.
[FIG. **12**] A flowchart showing a procedure of processing by a content analysis section **14** for analyzing a content in which video and audio are diversely present.
[FIG. **13**] A flowchart showing a procedure of processing by a content analysis section **14** for analyzing an audio content.
[FIG. **14**] A diagram showing a functional block construction of an electroencephalogram interface system **1** according to Embodiment 3.
[FIG. **15**] A flowchart showing the procedure of a process of analyzing changes in an environment concerning sounds.

### DESCRIPTION OF THE REFERENCE NUMERALS

- **1**: electroencephalogram interface system
- **2,50**: electroencephalogram distinction method adjustment apparatus
- **3**: CPU
- **4**: RAM
- **5**: computer program
- **11**: output section
- **12**: biological signal measurement section
- **13**: electroencephalogram interface section
- **14**: content analysis section
- **15**: electroencephalogram storage section
- **16**: user characteristic extraction section
- **17**: distinction method adjustment section
- **51**: external environment detection section
- **52**: external environment analysis section

### BEST MODE FOR CARRYING OUT THE INVENTION

Hereinafter, with reference to the attached drawings, embodiments of an electroencephalogram interface system and an electroencephalogram distinction method adjustment apparatus to be incorporated in the electroencephalogram interface system according to the present invention will be described.

First, main features of the electroencephalogram interface system and electroencephalogram distinction method adjustment apparatus according to the present invention will be outlined. Thereafter, each embodiment of the electroencephalogram distinction method adjustment apparatus will be described.

The inventors envisage that, in future, an electroencephalogram interface system will be constructed in an environment in which a wearable-type electroencephalograph and a wearable-type display are combined. The user will always be wearing the electroencephalograph and the display, and be able to perform content viewing and screen manipulation by using the wearable-type display. Otherwise, it is envisaged that an electroencephalogram interface system will be constructed in an environment (e.g., home) in which a home television set and a wearable-type electroencephalograph are combined. When watching television, the user is able to perform content viewing and screen manipulation and the like by wearing the electroencephalograph.

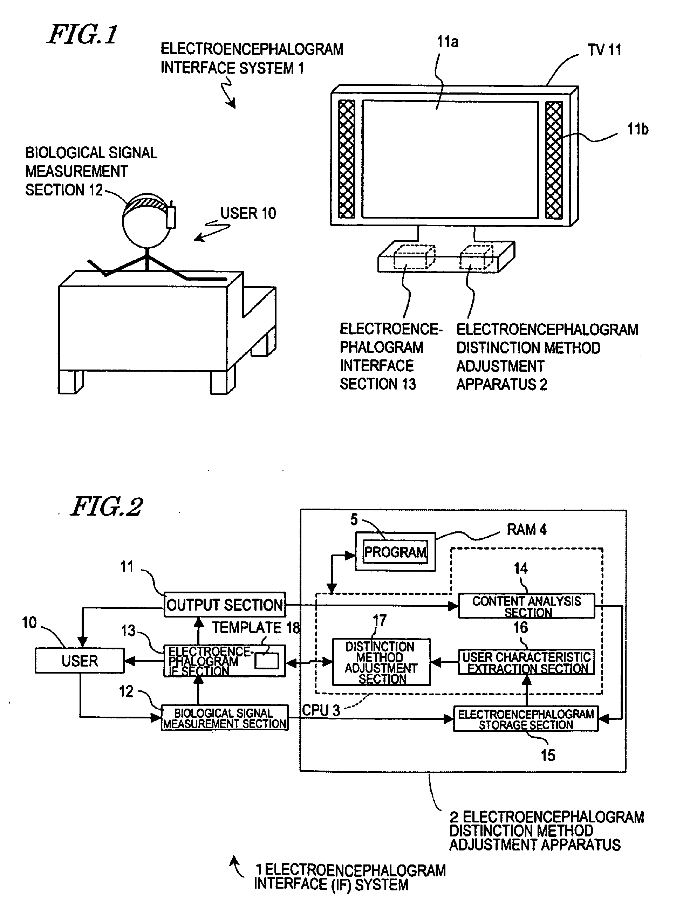

For example, FIG. **1** illustrates a construction and an environment of use for the electroencephalogram interface system **1** as envisaged by the inventors in the latter example. The electroencephalogram interface system **1** is exemplified so as to correspond to a system construction of Embodiment 1 described later.

The electroencephalogram interface system **1** is a system for providing an interface for manipulating a TV **11** by utilizing an electroencephalogram signal from a user **10.** An electroencephalogram signal from the user **10** is acquired by a biological signal measurement section **12** which is worn on the head of the user, and transmitted to an electroencephalogram interface section **13** in a wireless or wired manner. The electroencephalogram interface section **13** internalized in the TV **11** recognizes an intent of the user by utilizing a P3 component of an event-related potential which constitutes a part of the electroencephalograms, and performs processes such as channel switching.

The "P3 component" refers to a positive component of the event-related potential which appears in a time slot of 250 milliseconds to 500 milliseconds after a target stimulation is presented, regardless of the type of sensory stimulation such as auditory sense, visual sense, or somatic sensation. Typically, it refers to a positive component which appears near 300 milliseconds after a target stimulation is presented. In the descriptions of the following embodiments, the P3 component of an event-related potential occurring due to a stimulation to vision may be expressed as a "visual P3 component" and so on.

The time at which a P3 component in the electroencephalogram signal (event-related potential) may actually appear and its amplitude may fluctuate from user to user. Therefore, in the electroencephalogram interface system **1,** it is necessary to adjust the operating criterion in accordance with the electroencephalogram signal from the user **10.** The process for acquiring a criterion for performing this adjustment is the so-called calibration. The calibration is performed by the electroencephalogram distinction method adjustment apparatus **2.**

In the electroencephalogram interface system **1,** it is possible to measure electroencephalograms even in states other than when using the electroencephalograms as an interface, and so electroencephalograms can be measured even during viewing of a content such as television or a movie.

While the user is viewing a content, the electroencephalogram distinction method adjustment apparatus **2** collects data which is necessary for calibration. More specifically, the electroencephalogram distinction method adjustment apparatus **2** analyzes the data of the content being output, detects a change in the content that affects the event-related potential, and stores the waveform of an event-related potential during a predetermined period whose starting point is the point in time at which the change is detected. For example, a change in the content is detected when the luminance or hue of a moving picture being presented on a screen **11a** of the TV **11** changes beyond a threshold, or when the level of the audio being output from loudspeakers **11b** of the TV **11** changes beyond a threshold. Such a change in the content is considered as a change that affects the event-related potential.

The collected data is used for calibration when the user manipulates a device such as the TV **11** by utilizing electroencephalograms. However, since the necessary data has already been collected, no explicit calibration process is presented to the user, and the user will not recognize that a calibration process is under way. Since the calibration is achieved with respect to each user, every user is able to accurately manipulate a device such as the TV **11** without using a hand, even in the case where their both hands are full due to a household chore or rearing of children, for example. Thus, the manipulability of the device is significantly improved.

In accordance with the electroencephalogram interface system **1** as such, when starting use of the device, the user does not need to perform any electroencephalogram measurement that is solely directed to calibration, whereby the burden and trouble to the user at the time of starting use of the device are eliminated. Since no time needs to be spent for calibration when starting use of the system **1**, the user is able to immediately start viewing a content. Given the fact that use of the device is supposed to be started for the purpose of content viewing or the like, it is very useful that an operation which accords to the desire of the user can be immediately realized.

Note that, in the present invention, changes in a stimulation which is given to the user are detected in order to acquire information concerning individual differences which are necessary for calibration of the electroencephalogram interface system. Changes in the stimulation are detected as follows. That is, a physical quantity corresponding to a visual and/or auditory stimulation is measured and analyzed as a characteristic quantity of the stimulation, and by detecting a change in the characteristic quantity of the stimulation, a change in the stimulation is detected.

However, the specific method of detecting a change in the stimulation differs between Embodiments 1 and 2 and Embodiment 3.

In Embodiments 1 and 2, while the user is viewing a video/audio content or an image, based on a change in a stimulation (video and/or audio) which the user has received from the device and on a change in an event-related potential caused by that change, the change in the stimulation is detected.

On the other hand, in Embodiment 3, in an environment within which the user is situated, based on a change in a stimulation (ambient light and/or environmental sound, etc.) which the user has received from the environment and on a change in an event-related potential caused by that change, the change in the stimulation is detected.

Hereinafter, Embodiments 1 to 3 of the present invention will be respectively described.

### (Embodiment 1)

FIG. **2** shows a functional block construction of the electroencephalogram interface system **1** according to the present embodiment. The electroencephalogram interface system **1** includes the electroencephalogram distinction method adjustment apparatus **2,** an output section **11,** the biological signal measurement section **12,** and the electroencephalogram interface (IF) section **13.** FIG. **2** also shows detailed functional blocks of the electroencephalogram distinction method adjustment apparatus **2.** The user **10** block is illustrated for convenience of explanation.

The electroencephalogram distinction method adjustment apparatus **2** is connected to each of the output section **11,** the biological signal measurement section **12,** and the electroencephalogram interface section **13** in a wired or wireless manner, and performs transmission and reception of signals. Although FIG. **1** illustrates the electroencephalogram interface section **13** and the electroencephalogram distinction method adjustment apparatus **2** as separate entities, this is only exemplary. Some or all of them may be integrated.

The electroencephalogram distinction method adjustment apparatus **2** is provided in order to calibrate the electroencephalogram interface section **13** based on an electroencephalogram signal from each user in the electroencephalogram interface system **1**. A detailed description of the electroencephalogram distinction method adjustment apparatus **2** will be set forth later.

The output section **11** outputs to the user a content and a menu to be selected in the electroencephalogram interface. Since the TV **11** shown in FIG. **1** is a specific example of the output section, reference numeral **"11"** will hereinafter be assigned to the output section. The output section **11** would correspond to the display screen **11a** (FIG. **1**) in the case where the output content is a moving picture or a still image, and correspond to the loudspeakers **11b** (FIG. **1**) in the case where the output content is audio. Note that the display screen **11a** and the loudspeakers **11b** may be together used as the output section **11.**

The biological signal measurement section **12** is an electroencephalograph which detects a biological signal by measuring a change in potential on electrodes which are worn on the head of the user **10,** and measures electroencephalograms as a biological signal. The electroencephalograph may a head-mounted electroencephalograph as shown in FIG. **1****.** It is assumed that the user **10** has put on the electroencephalograph in advance.

Electrodes are disposed on the biological signal measurement section **12** so that, when worn on the head of the user **10,** the electrodes come into contact with the head at predetermined positions. The positioning of the electrodes may be, for example, Pz (median vertex), A1 (earlobe), and the root of nose of the user **10.** However, it will suffice if there are at least two electrodes, and potential measurement will be possible with only Pz and A1, for example. These electrode positions are to be determined based on reliability of signal measurements, wearing ease, and the like.

Thus, the biological signal measurement section **12** is able to measure the electroencephalograms of the user **10.** The measured electroencephalograms of the user **10** are sampled so as to be computer-processable, and are sent to the electroencephalogram interface section **13** and the electroencephalogram distinction method adjustment apparatus **2.** Note that, in order to reduce the influence of noises which may be mixed in the electroencephalograms, the electroencephalograms to be measured in the biological signal measurement section **12** are subjected to band-pass filtering from e.g. 0.05 to 20Hz in advance, and to baseline correction with respect to an average potential at e.g. 200 milliseconds before a menu item or an auditory stimulation is presented.

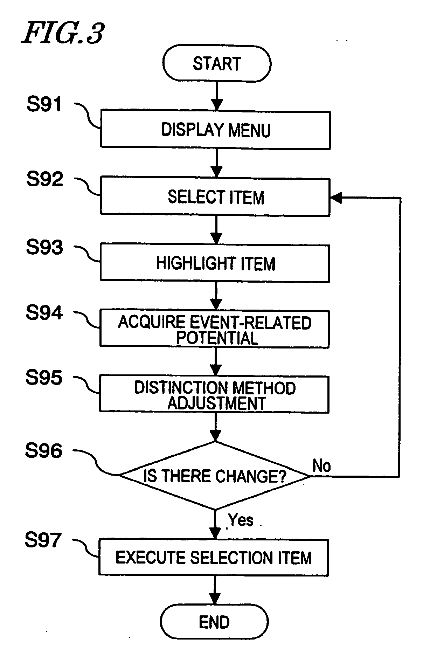

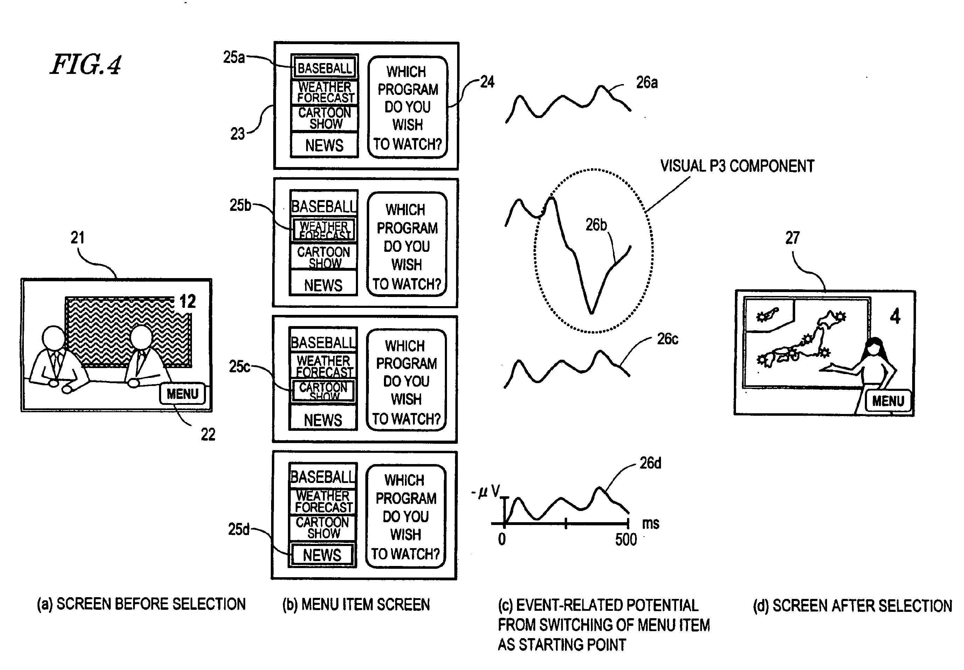

The electroencephalogram interface section **13** presents menu items concerning device manipulations to the user, cuts out an event-related potential of the electroencephalograms measured by the biological signal measurement section **12,** and subjects it to distinction. In making the distinction, the electroencephalogram interface section **13** uses a waveform template **18.** The template **18** defines, for example, data representing the waveform of an event-related potential of electroencephalograms which should appear when a desire is met. The electroencephalogram interface section **13** displays a menu item and the like via the output section **11,** and evaluates whether or not the waveform of an event-related potential acquired thereafter is close to the waveform of the template **18.** When it is evaluated to be close, the electroencephalogram interface section **13** instructs the device to execute an operation corresponding to that menu item. As a result, a change or correction of the content presented by the output section **11** becomes possible. Note that the details of the operation of the electroencephalogram interface section **13** will be described later with reference to FIG. **3** and FIG. **4****.**

The output section **11,** the biological signal measurement section **12,** and the electroencephalogram interface section **13** realize a main function of the electroencephalogram interface system **1**, i.e., an electroencephalogram interface function for providing device manipulation utilizing electroencephalograms.

Next, the construction of the electroencephalogram distinction method adjustment apparatus **2** will be described in detail.

The electroencephalogram distinction method adjustment apparatus **2** includes a CPU **3,** a RAM **4,** and an electroencephalogram storage section **15** such as an HDD.

The electroencephalogram storage section **15** receives an event-related potential of electroencephalograms from the biological signal measurement section **12,** and stores data of that waveform. The start and end of data storage take place at the timing with which storage instruction signals are received from a content analysis section **14** (CPU **3**) described later.

The RAM **4** retains a computer program **5.** By executing the program **5,** the CPU **3** performs various processes described later. When these processes are regarded functionwise, the respective processes are realized by the CPU **3** functioning as if a plurality of component elements. Within the CPU **3** in FIG. **2****,** the main processes to be performed by the CPU **3** are illustrated as three functional blocks. Specifically, the CPU **3** functions as the content analysis section **14,** a user characteristic extraction section **16,** and a distinction method adjustment section **17**. Hereinafter, the details thereof will be described.

The content analysis section **14** receives a content which is output to the output section **11,** and analyzes its substance. The analysis is performed from the standpoint as to whether any change in the content has occurred that affects an event-related potential of the user **10.** For example, it is known that the event-related potential is generally affected when the luminance or hue of a moving picture changes beyond a certain value. Therefore, based on the data of the content, the content analysis section **14** determines whether a change in the image characteristic quantity, such as luminance or hue of the moving picture, has become equal to or greater than a prescribed value (threshold). If it has become equal to or greater than the threshold, a storage instruction signal is output to the electroencephalogram storage section **15** because it is time to store the waveform of an event-related potential.

Since a storage instruction signal is output only when a situation with a large change in the content has occurred, the waveforms of event-related potential in similar situations are stored, which contain the characteristics of that user.

The user characteristic extraction section **16** extracts a characteristic contained in the electroencephalograms of the user **10** who is using the electroencephalogram interface system **1**. There are large individual differences in how electroencephalograms may come out. As used herein, individual differences in electroencephalograms mean characteristics in the waveforms of event-related potential, and more specifically, the shape, the amplitude level at a peak point, and the like.

The user characteristic extraction section **16** is provided in order to extract information of such an individual difference, i.e., a characteristic which is specific to the user. For example, the user characteristic extraction section **16** derives an arithmetic mean of the stored waveforms of event-related potential, and extracts a characteristic which is specific to the user.

Based on the information of an individual difference which has been extracted by the user characteristic extraction section **16,** the distinction method adjustment section **17** adjusts the data of the template **18** for distinction, which is to be utilized in the electroencephalogram interface section **13,** so that the user's characteristic becomes distinguishable. This makes it possible to maintain a high distinction ability in the electroencephalogram interface section **13** in spite of individual differences.

Note that the content analysis section **14,** the user characteristic extraction section **16,** and the distinction method adjustment section **17** mentioned above do not need to be realized by a single CPU **3,** but may be implemented in the form of respective process chips.

Next, with reference to FIG. **3** and FIG. **4****,** the main processes of the electroencephalogram interface system **1** shown in FIG. **2** will be generally described. Thereafter, the need for calibration will be described.

The electroencephalogram interface system **1** is provided for the purpose of using an event-related potential to distinguish which item the user wants to select from among a plurality of selection items displayed on the TV screen or the like.

FIG. **3** shows a procedure of processing by the electroencephalogram interface section **13.** On the other hand, FIGS. **4(a)** to **(d)** show an example where the TV in the electroencephalogram interface system **1** is manipulated for the user **10** to watch a program of a genre that the user wishes to view.

Hereinafter, according to the flowchart shown in FIG. **3****,** an operation of the electroencephalogram interface section **13** will be described while referring to FIG. **4** as necessary.

At step **S91,** the electroencephalogram interface section **13** displays a menu via the output section **11.** Assume for example that, as shown in FIG. **4(a)****,** a screen **21** before making a selection (i.e., news in this case) is being displayed on the TV display during content viewing. A "menu" **22** shown at the lower right is flickering at a specific frequency. When the user watches the "menu" **22,** a specific frequency component is superposed on his or her electroencephalograms, and thus it can be determined whether the "menu" **22** is being watched or not, and the electroencephalogram interface can be activated. To "activate" the electroencephalogram interface means to start the operation of an interface for enabling selection from a menu or the like by using electroencephalograms.

Once the electroencephalogram interface is activated, a menu item screen **23** as shown in FIG. **4(b)** is displayed. On the screen, a question **24** that says "Which program do you wish to watch?", and alternatives **25** which are candidates of a program that may be being desired for watching, are presented. In this example, four are being displayed: "baseball" **25a,** "weather forecast" **25b,** "cartoon show" **25c,** and "news" **25d.**

At step **S92,** the electroencephalogram interface section **13** selects one of the items. In the example of FIG. **4(b)****,** the baseball **25a,** which is the topmost, is first selected. Then, every time this step **S92** is executed, a next alternative is consecutively selected, until wrapping around to the topmost baseball after the fourth, i.e., news.

At step **S93,** the electroencephalogram interface section **13** highlights an item which is selected at step **S92.** Highlight is an indication using a background which is brighter than any other item or an indication in a bright text color. Note that, instead of or in addition to highlight, a point or cursor employing an auxiliary arrow may be used to point to an item. Herein, it suffices if, when the user **10** looks at it, it is clear which item the system is currently demanding attention to.

At step **S94,** the electroencephalogram interface section **13** acquires an event-related potential from the electroencephalograms having been measured by the biological signal measurement section **12.** The starting point at which to start acquisition of an event-related potential is set at the moment of highlighting at step **S93.** Then, an event-related potential from e.g. 200 milliseconds before and until 1 second after the moment is acquired. As a result, the user's response to the highlighted item is obtained.

At step **S95,** the electroencephalogram distinction method adjustment apparatus **2** adjusts the distinction method in the electroencephalogram interface section **13.** In the present embodiment, based on a characteristic of the user **10** as calculated by the user characteristic extraction section **16,** the distinction method adjustment section **17** adjusts the template **18** in the electroencephalogram interface section **13.** Through this process, an adjustment is made so as to arrive at a distinction method which supports the user's individual difference. The details of the user characteristic extraction and the distinction method adjustment will be described later.

At step **S96,** the electroencephalogram interface section **13** distinguishes the currently acquired event-related potential by using the template **18** after adjustment. The distinction is directed to whether the waveform of the currently acquired event-related potential is a waveform to appear when the user **10** is watching an item which he or she wishes to select or a waveform to appear when the user **10** is watching an item which he or she does not wish to select.

FIG. **4(c)** shows waveforms **26a** to **26d** of event-related potential which are acquired from the moments at which the respective menu items are highlighted. For example, assuming that the user **10** is wishing to watch the weather forecast, a characteristic component will appear only in the waveform **26b** when the weather forecast item is highlighted. This is a waveform component called a visual P3 component of an event-related potential, which is a positive characteristic waveform that appears about 300 milliseconds after the switching of the menu item. The electroencephalogram interface section **13** determines whether or not this component is observed in the waveform of the acquired event-related potential. If the visual P3 component is observed, control proceeds to step **S97.** If it is not observed, control returns to step **S92.**

At step **S97,** determining that the item for which the visual P3 component of the user **10** has been observed is the item which the user **10** wants to select, the electroencephalogram interface section **13** instructs the TV to execute a process corresponding to the selected item. In the example of FIG. **4(c)****,** since the visual P3 component is observed for the weather forecast item **25b,** the electroencephalogram interface section **13** instructs the TV to switch the channel so as to display the weather forecast content. As the TV switches the channel based on this instruction, the weather forecast is displayed on the screen **27** as shown in FIG. **4(d)****.**

Through the aforementioned process, without performing button manipulations or the like, the user selects a menu item based on electroencephalograms, and is able to view a content corresponding to the desired item.

Although it has been illustrated that the items are consecutively selected at step **S92,** a method of making random presentations would also be possible. This may lead to a possibility in that a menu selection may be made in a more careful manner because it is not known in advance which item will be selected.

In such an electroencephalogram interface, in order to realize a smooth manipulation which accords to the intent of the user **10,** a high distinction ability for the event-related potential will be required. However, since the electroencephalogram signal has large individual differences, it is difficult to perform distinction with a single criterion.

FIG. **5** shows examples of individual differences in electroencephalogram signals which are described in Non-Patent Document 1. FIG. **5** shows exemplary electroencephalogram signals from 36 people, concerning discrimination problems in response to visual stimulations. Non-Patent Document 1 describes that: there are three times as large individual differences in the amplitude of the event-related potential as there are in the response time; the amplitude range is from 4.4V to 27.7V (average 16.64 µV, standard deviation 6.17 µV); and the individual differences amount to larger differences than behavioral indices such as response time.

Therefore, unless it is grasped as to electroencephalograms of which characteristics are being produced by the user **10** who is using the electroencephalogram interface system **1,** smooth manipulations in the electroencephalogram interface system **1** cannot be realized. In other words, a calibration for adjusting the operating criterion to the electroencephalogram signal of each user **10** is necessary.

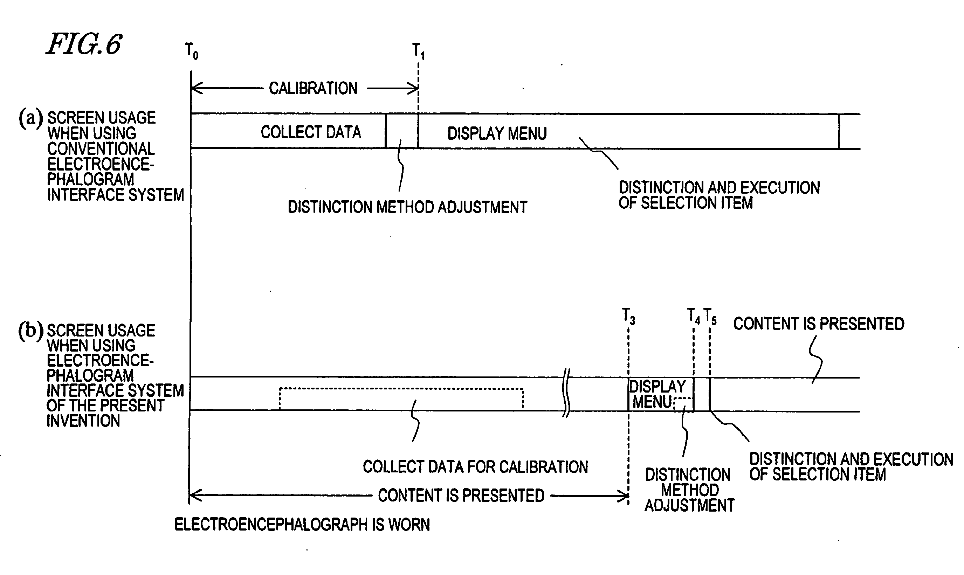

Therefore, in order to more clearly understand the features of the present invention, with reference to FIG. **6****,** calibration in a conventional electroencephalogram interface system such as research or medical applications and calibration in the electroencephalogram interface system **1** according to the present embodiment will be described in comparison.

In a conventional calibration, before use of the interface, it is first necessary to take measurements in a situation similar to using the interface. FIG. **6(a)** shows in chronological order a screen usage when performing a conventional calibration. The horizontal axis is time.

Until time **T1** from time **T0** (starting point) at which the electroencephalograph is worn, the screen is used for performing a calibration. Facing the screen, the user needs to produce an electroencephalogram signal for a manipulation based on electroencephalograms. After electroencephalogram signal data is collected, adjustment of the distinction method is made, and a message is displayed on the screen that the adjustment is being made.

After completion of the calibration, a menu is displayed on the screen, and a distinction between selection items using the electroencephalogram interface system is performed. For example, alternatives are consecutively highlighted, and an event-related potential at each time is measured to determine the user's desired alternative, and an operation (task) of the determined alternative is executed.

Note that, in the conventional techniques including research or medical applications, etc., the menu to be displayed on the screen is not a menu for selecting a content or the like, but is a menu listing candidates of tasks whose execution may be requested. An example of a task to be executed may be, when a patient requests something to drink in a hospital, a task for a nurse or the like to bring something to drink in accordance with the alternative, after the alternative is decided.

On the other hand, in the present embodiment, calibration is performed by using a characteristic of the user as calculated from the amplitude of an event-related potential during content viewing. The principles thereof are as follows.

Factors of the aforementioned individual differences may be anatomical individual differences (shape of the cranium or the brain), how the electrodes are worn, changes in the arousal level within each individual, differences in the manner of handling a problem, and the like.

Between the amplitude of the event-related potential during content viewing and the amplitude of the event-related potential with respect to each menu item when the electroencephalogram interface is activated, some correlation can be considered to exist as for anatomical individual differences, how the electrodes are worn, and changes in the arousal level, among the above fluctuations. The reason is that these will affect the shape of the electroencephalograms regardless of the type of problem. Therefore, by at least using a user characteristic which is calculated from the amplitude of an event-related potential during content viewing, it becomes possible to perform calibration and improve the distinction ability during use of the electroencephalogram interface.

FIG. **6(b)** shows in chronological order a screen usage when performing a calibration according to the present embodiment. The horizontal axis is time.

In the electroencephalogram interface system **1** according to the present embodiment, it is possible to immediately allow a content to be displayed from time **T0** at which the electroencephalograph is worn, or to perform a content selection or the like with the electroencephalogram interface. In the case of using the electroencephalogram interface from the beginning, the electroencephalogram interface may make a determination of an alternative based on a predetermined value. For example, the amplitude of a standard P3 component, the waveform of an event-related potential of that user from the previous time, or the like can be used. Although no particular use of the electroencephalogram interface is made during content viewing, calibration data is collected during content viewing.

Thereafter, when content viewing is ended at time **T3,** the menu shown in FIG. **4** is displayed. Then, while the menu is being displayed, or specifically, at the phase shown as step **S95** in FIG. **3****,** a calibration is executed inside the electroencephalogram interface system **1.** At time **T4,** displaying of the menu is ended, and thereafter distinction of a selection item using the electroencephalogram interface system **1** is performed, and a corresponding operation is executed. As a result, the selected content is presented after time **T5.**

According to the present embodiment, no period is explicitly provided for acquiring the data for calibration as is conventionally done, and therefore the user is able to immediately start content viewing. This is very useful to the user because the user's purpose for starting the use of the device is content viewing or the like.

Moreover, since calibration data is collected during content viewing and thereafter a calibration is performed, manipulability of the electroencephalogram interface is also improved. At this time, since the user is not aware of any explicit calibration process, it may be said that there is quite no burden on the user.

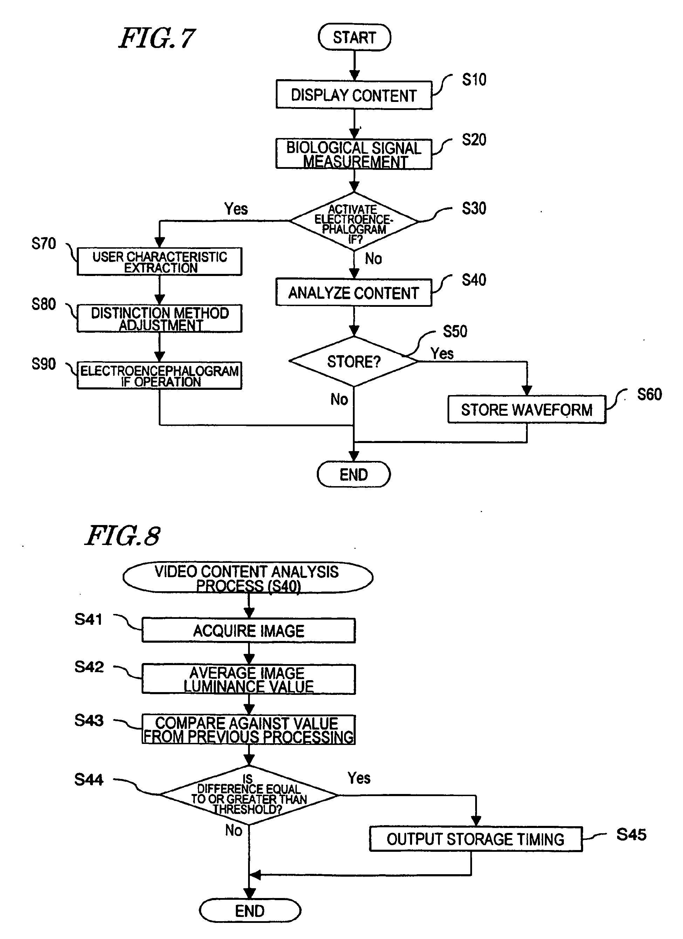

Next, with reference to FIG. **7****,** a procedure of processing by the electroencephalogram interface system **1** according to the present embodiment will be described. The following description assumes that, as in the example of FIG. **3****,** switching of the contents to be presented on the TV and changing of the channel and sound volume level are performed by using the electroencephalogram interface system **1.** The entire processing proceeds by repeating the processes from step **S10** to step **S90** below.

FIG. **7** shows a procedure of processing by the electroencephalogram interface system **1** of the present embodiment.

At step **S10,** the output section **11** displays a content. Usually, a content such as a television program or a movie is displayed on the TV screen.

At step **S20,** the biological signal measurement section **12** measures electroencephalograms. It is assumed that the electroencephalograph is worn both during content viewing and during use of the electroencephalogram interface.

At step **S30,** the electroencephalogram interface section **13** determines whether the user is desiring to activate the electroencephalogram interface or not.

When the desire to activate the electroencephalogram interface is confirmed, control proceeds to the characteristic extraction process of step **S70.** When it is not confirmed, control proceeds to the content analysis process of step **S40.**

SSVEP or P300 can be used for the determination as to whether there is a desire to activate the electroencephalogram interface or not. SSVEP means Steady State Visual Evoked Potential.

As has been described in connection with FIG. **4(a)****,** on the screen before selection, a "menu" **22** is displayed at the lower right, the "menu" **22** flickering at a specific frequency. When the user sees the "menu" **22,** a specific frequency component is superposed on his or her electroencephalograms, and thus it can be determined whether the "menu" **22** is being watched or not, and the electroencephalogram interface can be activated. Note that an accurate operation even before calibration is possible because the presence or absence of a desire to activate the electroencephalogram interface can be determined based on whether the specific frequency component is superposed on the electroencephalograms or not.

For detail of SSVEP, see Xiaorong Gao, et al., "A BCI-Based Environmental Controller for the Motion-Disabled", IEEE Transaction on Neural Systems and Rehabilitation Engineering, Vol.11, No.2, June 2003.

From step **S40** to step **S60,** since it has been confirmed at step **S30** that the electroencephalogram interface is not meant to be activated, a content analysis is performed as a preparatory process for grasping a characteristic of the electroencephalograms of the user **10.**

At step **S40,** the content analysis section **14** analyzes the data of the content, and identifies a scene included in the content where a characteristic on the electroencephalograms of the user **10** is likely to appear.

In the electroencephalogram interface system **1,** a request of the user is determined by using an event-related potential waveform which occurs when the menu is highlighted. Therefore, during content viewing, the content analysis section **14** extracts scenes in which similar signals are likely to be observed. For example, it is considered that a component similar to the visual P3 component can be observed in a scene where there is a large change in the luminance of the screen. Therefore, relative to a predetermined threshold, the content analysis section **14** makes an analysis as to whether there is any content scene with a large change in the luminance of the screen, based on the content data.

At step **S50,** the content analysis section **14** determines whether or not to store the current electroencephalogram. As a result of the analysis of step **S40,** if it is determined that the current electroencephalogram includes a trend which is similar to that of the electroencephalograms while the user is using the electroencephalogram interface, i.e., if it is determined there is a large change in the luminance of the screen, control proceeds to step **S60.** If it is determined that there is no large change in the luminance of the screen, the process is ended.

At step **S60,** the electroencephalogram storage section **15** stores electroencephalograms (event-related potential) around the timing to store electroencephalograms which is determined through the content analysis. In the electroencephalogram interface system **1**, it is necessary to catch a change in the waveform of the event-related potential from a specific point in time. Since it is necessary to have a uniform baseline for determination, the electroencephalogram storage section **15** cuts out - 200 milliseconds to 1000 milliseconds from the storage timing and stores it, for example. Through such storage and summation, a characteristic of the user can be extracted while reducing shifts in the electroencephalograms each time and noise influences.

Step **S70** to step **S90** are executed in the case where activation of the electroencephalogram interface is desired at step **S30**. In these processes, preparations to execute the electroencephalogram interface and an operation of the electroencephalogram interface are performed.

At step **S70**, the user characteristic extraction section **16** extracts a characteristic of the event-related potential waveform of the user who is currently wearing the electroencephalograph. For example, the user characteristic extraction section **16** may take an arithmetic mean of the event-related potential waveform data stored in the electroencephalogram storage section **15,** and extract a characteristic of the electroencephalograms. A characteristic of the electroencephalograms may be, for example, a trend in the amplitude level of the waveform of the user.

At step **S80,** the distinction method adjustment section **17** adjusts a distinction method to be adopted in the electroencephalogram interface section **13.** This process is performed based on a characteristic quantity which is extracted at step **S70.** The process of adjusting the distinction method will be described later.

At step **S90,** the electroencephalogram interface section **13** provides the function of the electroencephalogram interface. The specific processes are as have been described with reference to FIG. **3** and FIG. **4****.**

Through the above processes, characteristics pertaining to an individual difference of the user are stored during content viewing, when the electroencephalogram interface is not activated. Once the electroencephalogram interface is activated, the distinction method in the electroencephalogram interface section **13** is adjusted by using the stored data of the user's individual difference characteristic. As a result, an ability to accurately distinguish electroencephalograms having large individual differences is provided.

Next, each of step **S40,** step **S70,** and step **S80** above will be described in further detail with reference to FIG. **8** to FIG. **11****.**

First, with reference to FIG. **8****,** details of the content analysis process described at step **S40** in FIG. **7** will be described. In the description, it is assumed that the content is a moving picture.

FIG. **8** shows a procedure of a process of analyzing a video content which is a moving picture.

At step **S41,** the content analysis section **14** acquires data of the video content. For example, the content analysis section **14** acquires still image data by capturing a video which is currently being displayed. As is well-known, a moving picture is constituted by displaying a plurality of still pictures which are switched one after another at a predetermined frequency (e.g., 30Hz). By capturing a moving picture at a given moment, a single still image data will be obtained.

Moreover, instead of acquiring still image data from the video which is currently being displayed, it is also possible to acquire still image data based on the moving picture data for displaying that video. For example, since digitized moving picture data is distributed in a digital broadcast, the still image data composing the moving picture can be extracted from that moving picture data.

At step **S42,** the content analysis section **14** calculates an average of image luminance values. Images themselves contain image characteristic quantities such as luminance, hue, and chroma, and various information such as the number of pixels. In the present embodiment, in order to know an overall trend in the visual stimulations to the user **10**, an average of the luminance values of images is used, and behavior of the changes in this average value is examined.

At step **S43,** the content analysis section **14** compares between the average value calculated at step **S42** and the average value from the previous processing. As a result, it becomes possible to makes a determination as to whether there is a large change in luminance. Note that, when conducting a comparison for the first time, any arbitrary value may be used as an "average value from previous processing", or average values may be ascertained two times, and the average value from the first time may be adopted as the "average value from previous processing".

At step **S44,** the content analysis section **14** determines whether or not the amount of change in luminance at step **S43** is equal to or greater than a predetermined threshold. If it is equal to or greater than the threshold, step **S45;** if it is not equal to or less than the threshold, the process is ended.

At step **S45,** determining that the waveform of the event-related potential of the electroencephalograms must be stored from that point in time, the content analysis section **14** outputs to the electroencephalogram storage section **15** a storage instruction signal indicating storage timing. The reason is that, in a situation where step **S45** is executed, it is presumable that an event-related potential to a stimulation has been induced in the user's electroencephalograms and thus there is need to store the data. As a result, the waveform of an event-related potential is stored which represents the characteristic of the user in the neighborhood of a scene with a large change in the luminance of the screen.

Note that, in the case of the television screen, a scene with a large change in the luminance of the screen may exist when a dark scene has suddenly changed into a bright scene in a movie or the like, or a television program has been switched to a CM, and so on. Being regarded as visual stimulations, these scenes constitute scenes which are likely to induce an evoked potential in the user **10**. By extracting only such situations, it becomes possible to obtain an effect similar to data collection in a laboratory room for measuring response to visual stimulations.

Note that detection of a change in the luminance of images does not need to be performed with respect to each single frame. A magnitude of change across a plurality of frames may be taken into account. There is no problem in not using an average of luminance, so long as the trend in the event-related potential can be grasped and there is a characteristic visual stimulation to the user.

Note that the timing to store a waveform of event-related potential may be determined based on the appearance of an image that has the same luminance or little change in luminance but has a large change in hue. Thus, storage timing may be determined by utilizing hue alone. It would also be possible to utilize chroma or other image characteristic quantities.

The stored amount in the electroencephalogram storage section **15** must be determined based on considerations such as: a sufficient amount should be stored; excessively old data should not be used; and so on. In the studies of event-related potential, about 20 times of data summation is required, generally speaking. Therefore, for accuracy's sake, it is preferable to store data about 20 times.

Moreover, it is known that the amplitude of electroencephalograms may change with lapse of time even under the condition of being worn by the same individual. Possibly, a piece of data which has spent a considerable amount of time may no longer be a useful piece of information for the sake of distinction. Therefore, the electroencephalogram storage section **15** may operate so as to store only the most-recent 20 times of data, or discard data which has spent a predetermined time (e.g., 1 to 2 hours). In the subsequently-described processes, out of the data stored in the electroencephalogram storage section **15,** the most-recent 20 times of data may be assigned for use. Then, once the electroencephalograph is detached from the user and is worn again, the data up to then may be discarded, and data storage may be started anew.

As a result of the above processing, appropriate event-related potential waveforms are stored in the electroencephalogram storage section **15.**

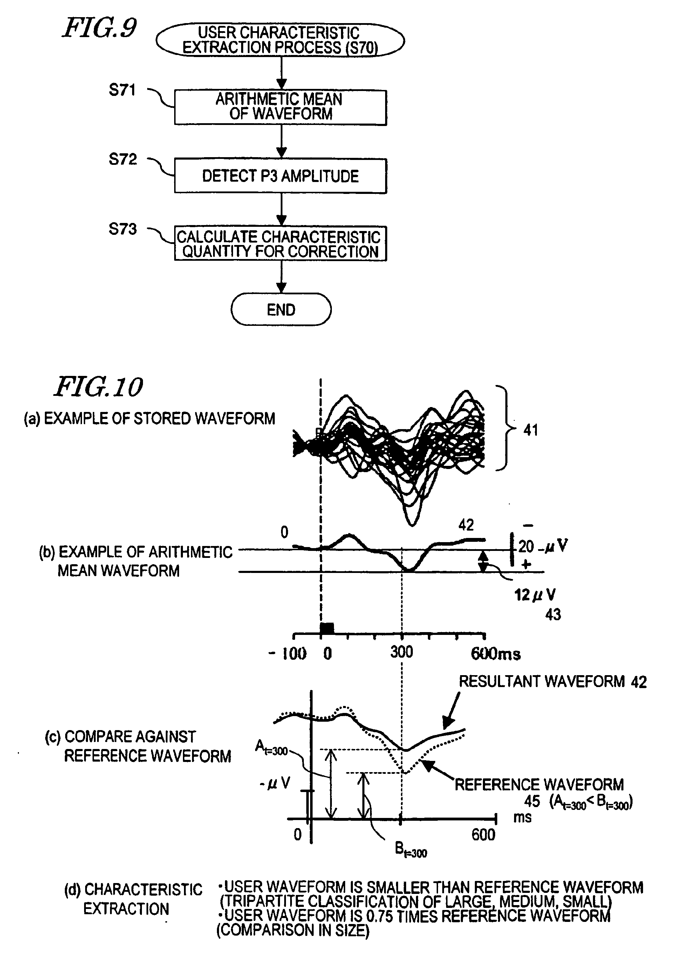

Next, with reference to FIG. **9** and FIG. **10****,** details of the extraction process for user characteristics stated at step **S70** in FIG. **7** will be described. In the description, it is assumed that the content is a moving picture.

FIG. **9** shows a procedure of an extraction process for a characteristic of a user based on the waveform of an event-related potential. FIGS. **10(a)** to **(d)** show examples of waveforms associated with a process of extracting a characteristic of a user. First, as shown in FIG. **10(a)****,** it is assumed that a plurality of event-related potential waveforms **41** are stored in the electroencephalogram storage section **15.** This is an example shown in Non-Patent Document 1, which is hypothetically presented herein. Since the event-related potential waveforms **41** were extracted upon appearance of scenes with large changes in luminance, they have been stored under similar conditions. Under this premise, the processing by the user characteristic extraction section **16** shown in FIG. **9** is started.

At step **S71,** the user characteristic extraction section **16** calculates an arithmetic mean of the event-related potential waveforms **41** stored in the electroencephalogramelectroencephalogram storage section **15**. As a result, various influences such as electro-oculographic potentials and background electroencephalograms are reduced, so that only the component of the event-related potential that is of interest is emphasized and becomes easier to see. FIG. **10(b)** illustrates a waveform **42** which is an arithmetic mean. While FIG. **10(a)** reveals waveforms **41** which have been stored under similar conditions but do not allow for any clear-cut determination of a characteristic, FIG. **10(b)** may be said to present a characteristic alone, in a manner which is easy to see.

At step **S72** in FIG. **9****,** the user characteristic extraction section **16** detects (calculates) a P3 amplitude for the arithmetic mean waveform calculated at step **S71.** This "P3 amplitude" refers to a peak potential of the P3 component of the event-related potential. Given that the P3 component is a positive component which is observed near 300 milliseconds from a starting point of an event-related potential, it is the positiveness peak potential thereof near 300 milliseconds.

In the example of FIG. **10(b)****,** a lower (positive) potential peak is observed in the arithmetic mean waveform **42** near 300 milliseconds. A level **43** of the potential at this position was measured to be 12 µV. Since this amplitude has a large individual difference, this amplitude is to be utilized as a characteristic of each user, which defines the concept of the present invention.

It is possible to prescribe an average magnitude of amplitude. For example, in the example of Non-Patent Document 1, the average was 16.64 µV. In order to be more accurate, reference values corresponding to changes in images may be calculated in advance.

At step **S73** in FIG. **9****,** the user characteristic extraction section **16** determines whether the amplitude **43** of the P3 component calculated at step **S72** is greater or smaller than the amplitude of an expected average P3 waveform **45.** Then, a characteristic quantity for correction purposes is calculated.

For example, the amplitude **43** of the P3 component calculated at step **S72** can be classified and characterized into three kinds, i.e., similar level to the average value, greater than the average value, or smaller than the average value.

The classification is performed because it is necessary for calculating a characteristic quantity for correction purposes. A characteristic quantity for correction purposes is determined in accordance with the classification. In other words, the characteristic quantity for correction purposes corresponds to a group name in grouping such as large, medium, small. As for degree of precision, this classification may be made even finer, depending on the purpose and measurement accuracy. It would also be possible to make a determination based on an amount relative to an average amplitude.

In FIG. **10(c)****,** a solid line **42** shows the resultant waveform, and a broken line **45** shows an average waveform (reference waveform). Classification based on comparison of their sizes is exemplified in FIG. **10(d)****.** A characteristic may be described in a tripartite classification such as "smaller than average waveform", or, assuming that the average amplitude is 16 µV and the amplitude of this time is 12 µV, their ratio of 0.75 may be designated a characteristic quantity for correction purposes.

Thus, a characteristic quantity which reflects the user's individual difference can be calculated based on waveforms which are stored during content viewing. Herein, an individual difference reflects not only the amplitude level of the waveforms of each individual, but also the manner of wearing in each time, a difference in arousal level at that time, and the like, such that all factors affecting their distinction are included in the characteristic quantity for correction purposes. Note that, from the waveforms of the aforementioned event-related potential of the user during content viewing, a variance in the average value per unit interval may be calculated, and their variance may be utilized.

Next, it will be described how the distinction method adjustment section **17** adjusts the distinction method in the electroencephalogram interface section **13** by utilizing a characteristic quantity which is calculated by the user characteristic extraction section **16.**

In the processing by the electroencephalogram interface section **13,** in order to determine whether a currently highlighted menu item is selected or not, a method called template matching, which utilizes templates, may be possible. For example, the electroencephalogram interface section **13** retains in advance a standard event-related potential waveform (A) when a menu item is selected, and a standard event-related potential waveform (B) when no menu item is selected, as templates **18.** Then, by distinguishing whether the currently observed event-related potential waveform is closer to template (A) or template (B), the electroencephalogram interface section **13** is able to determine whether the currently highlighted menu item is selected or not.

In the case where the amplitude level of the event-related potential of the current user **10** is standard, a simple matching against a reference template would enable distinction based on template matching. However, in the case where the current user **10** deviates from the standard amplitude, it becomes necessary to correct the template. Therefore, the template is corrected with a characteristic quantity which is calculated by the user characteristic extraction section **16.**

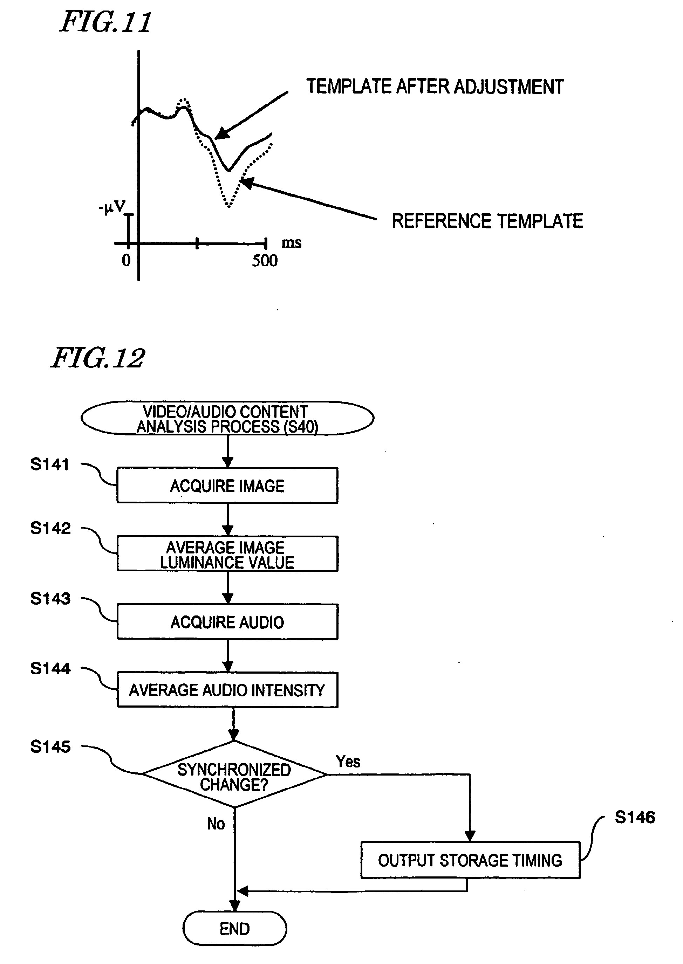

In the case where the user characteristic is classified into large, medium, small or the like, a template corresponding to each may be prepared, and such templates may be switched. Moreover, in the case where the user characteristic is expressed as a magnification such as 0.75, the potential may be multiplied by a predetermined factor (e.g., 0.75 in the above example) for each time slot of the reference template, whereby templates after adjustment can be generated. FIG. **11** shows a template before adjustment and a template after adjustment.

In the case where the individual difference appears greater in some time slots of the template waveform but smaller in others, that may also be taken into account for the template adjustment, in order to further enhance the accuracy. For example, in the case where the individual difference emanates from the electrodes being poorly worn or from the head shape or the like, it is possible that there may be an overall influence of amplitude variations. However, in the case where the cognitive arousal has lowered, care may be taken so that, for example, the characteristic quantity is less reflected before 200 milliseconds, where much component is presumed to directly respond to visual stimulations, and that the characteristic quantity is more reflected at 200 milliseconds to 500 milliseconds, where the P3 component appears well.

The above-described correction envisages changing of the template. Note however that the template may be left intact, and the amplitude of the event-related potential may be amplified or attenuated at the output from the biological signal measurement section **12** or at the input to the electroencephalogram interface section **13.** The amplitude of the event-related potential can be amplified or attenuated by the distinction method adjustment section **17** instructing the electroencephalogram interface section **13** or the biological signal measurement section **12.**

With such a construction, in accordance with the electroencephalogram distinction method adjustment apparatus of the present invention, information which is necessary for calibration is acquired while the user is viewing a content, without performing any explicit calibration. Therefore, an accurate determination of electroencephalograms is possible without the user having to spend time for calibration, thus providing an interface which is easy to manipulate.

In the above description, the content to be presented is a moving picture, and no particular mention of audio is made. However, during viewing of television or the like, it is likely that audio information is presented through loudspeakers or the like while video is being displayed on the display.

It is known that an event-related potential may also be induced in response to auditory stimulations, e.g., audio, as well as in response to visual stimulations, e.g., video. Therefore, it is conceivable that evoked potentials from various audio information may also be superposed on an event-related potential which has been selected based on a criterion from visual information alone, and such evoked potentials might be regarded as noises from the standpoint of distinction method adjustment. In order to more accurately measure a characteristic of the event-related potential that is related to an individual difference, an improvement in accuracy is expected by allowing not only an image analysis but also an audio analysis result to be taken into consideration at the content analysis section **14.**

Hereinafter, with reference to FIG. **12****,** the processing by the content analysis section **14** in the case where audio is output, together with video, from the output section **11.**

FIG. **12** shows a procedure of processing by the content analysis section **14** for analyzing a content in which video and audio are diversely present.

At step **S141,** the content analysis section **14** acquires image data which is being presented by the output section **11** at that point in time, and at step **S142,** calculates an average value of the luminance of the acquired image data.

On the other hand, at step **S143,** the content analysis section **14** acquires audio data which is output from the output section **11,** and at step **S144,** calculates an average value of the audio level which is output based on the acquired audio data.

At step **S145,** the user characteristic extraction section **16** determines whether it is good timing for acquiring an event-related potential which well reflects an individual difference of the user. Specifically, when the aforementioned change in the image occurs and also a change in the audio is observed that is in synchronization with this change, it is determined that an event-related potential is acquirable which well presents the user's characteristic in response to a change in the stimulation to the user.

Changes in each of the image and the audio can be detected by the already-described processing method. By determining whether such changes are in synchronization or not, it becomes possible to utilize the amplitude of an event-related potential of the user as information concerning individual differences, without separating the evoked potential for the image from the evoked potential for the audio. If the image and the audio are determined as changing in synchronization at step **S145,** control proceeds to step **S146;** if they are not changing so, the process is ended.

At step **S146,** determining that an event-related potential to a stimulation is induced in the electroencephalograms of the present time, the user characteristic extraction section **16** determines a need to store data, and outputs this point in time as storage timing.

As described above, synchronized changes in the video and the audio are likely to occur corresponding to switching of scenes on television, and therefore data collection is well possible. Note that the processing order between steps **S141** and **S142** and steps **S143** and **S144** may be changed.

According to the above-described processes, information which is necessary for calibration can be obtained based on the magnitude of a change in the event-related potential that is caused by a change in a characteristic quantity of the image and/or audio, and more generally, based on the magnitude of a change in the event-related potential that is induced by a change in a characteristic quantity of a content.

The electroencephalogram storage section **15** extracts a stimulation only if an event-related potential in response to the stimulation is observed. However, it would also be possible to store data of conditions that result in little stimulation (i.e., little change in the screen). By doing so, it becomes possible to store both the electroencephalograms in the presence of stimulations and the electroencephalograms in scarcity of stimulations, and by using a difference therebetween, a difference of characteristics as to usually present electroencephalograms from the electroencephalograms in response to stimulations can be clearly acquired.

Note that the amplitude of the waveforms of electroencephalograms stored in the electroencephalogram storage section **15** may be checked at the electroencephalogram storage section **15** per every storage, and if the stored electroencephalograms are so small that they go beyond the usual range of individual differences, an output can be made from the output section **11** for prompting that the manner in which the electroencephalograph is worn needs to be checked. For example, in a situation where electroencephalograms are not being measured because of a poor manner of wearing the electroencephalograph when it was first worn, a message such as "electroencephalograms are not being measure yet. Wear it again" can be output. Furthermore, if a mechanism is provided for regularly checking the data in the electroencephalogram storage section, it becomes possible to detect cases where the manner of wearing the electroencephalograph has changed due to a body motion or the like and the signal has become weak, etc.; and even in such cases, a message such as "Please check how electroencephalograph is worn" can be output. This can realize a situation which permits natural use of the electroencephalogram interface, while preventing a situation where one notices that the electroencephalogram interface is not adequately usable only after the electroencephalogram interface is activated.

### (Embodiment 2)

Embodiment 1 has described a case where data for calibration is collected by mainly utilizing changes in images which are presented on a screen, thus adjusting the distinction method. In the present embodiment, a method for executing audio-based calibration for an audio-based interface will be described. When such a system is applied as a car navigation system for an automobile, for example, the user is enabled to manipulate the system even during driving, thus leading to a very high convenience.

In the present embodiment, only an audio channel is used when a content such as the radio is enjoyed, which is provided via broadcast or streaming. The basic construction of the electroencephalogram interface system **1** is as shown in FIG. **1****,** except that only loudspeakers may be provided instead of a TV.

At the output section **11,** an audio content is presented via loudspeakers or the like. The electroencephalogram interface is provided via a screen, or provided by sequentially reading aloud menu items in the form of audio. In the latter case, an electroencephalogram interface relying only on interactions through audio is constituted.

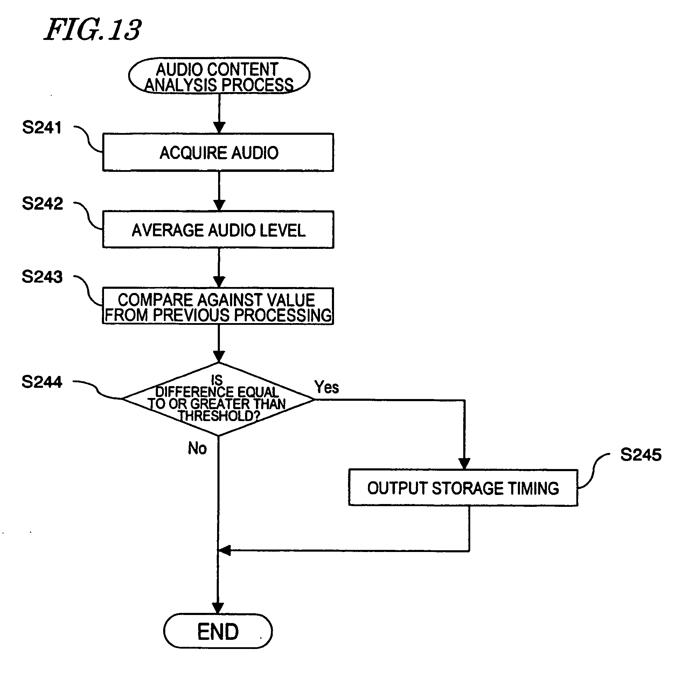

Hereinafter, with reference to FIG. **13****,** the processing by the content analysis section **14** in the case where an audio content is output from the output section **11** will be described.

FIG. **13** shows a procedure of processing by the content analysis section **14** for analyzing an audio content.

At step **S241,** the content analysis section **14** acquires audio data. The range of audio data to be acquired is an audio interval to follow an audio interval which was acquired in a previous process. Each audio interval can be defined based on a description based on time, e.g., sampling at every predetermined time, or a description based on signal intensity, e.g., detection of silent intervals.

At step **S242,** the content analysis section **14** calculates an average value of audio level of the audio data of the audio interval acquired.

At step **S243,** the content analysis section **14** makes a comparison between the average value of audio level as calculated in the previous time and the average value of this time. As a result, an interval which has experienced a large change in audio level can be detected.

At step **S244,** the content analysis section **14** determines whether or not the amount of change in audio level detected at step **S243** is equal to or greater than the predetermined threshold. If it is equal to or greater than the threshold, control proceeds to step **S245;** if it is equal to or less than the threshold, the process is ended.

At step **S245,** the content analysis section **14** determines a need to store data, and outputs this point in time as storage timing. The reason is that it is believed that an event-related potential to an audio stimulation is induced in the user's electroencephalograms.

Through the above operation, in an electroencephalogram interface mainly directed to audio, too, an event-related potential representing a characteristic of the user is induced when there is a large change in audio, and therefore this timing can be detected and its waveform can be stored in the next storage section **15.**

Now, examples of points associated with a large change in audio may be, in the audio track of television or on the radio: as for sports, sudden outburst of cheers following after a shot was scored during live soccer coverage; an abruptly-occurring big laugh in a comic show program or the like; and a time signal (e.g., "pop, pop, pop, beep"). By storing the event-related potential at such timing, responses to auditory stimulations can be collected.

With the electroencephalogram distinction method adjustment apparatus of the present embodiment, even in an electroencephalogram interface centered around audio, information which is necessary for calibration is acquired while the user is enjoying a content, without performing any explicit calibration. Therefore, an accurate determination of electroencephalograms is possible without requiring the user to spend time for calibration, thus providing an interface which is easy to manipulate.

### (Embodiment 3)

Embodiments 1 and 2 have illustrated examples of acquiring calibration information from contents such as television.

However, even during viewing of a content such as television, the electroencephalograms of the user **10** may be affected by factors other than the content. Examples of factors other than the content may be changes in the external environment. Specifically, a "slam" of a closing door, a time signal of a clock, a "ding dong" of an entry chime from a visiting guest, and the like may possibly also affect the electroencephalograms of the user **10.**

The inventors have found that, by sensing a stimulation from an external environment (i.e., light and/or sound in the environment within which the user exists), it is possible to acquire data for calibration from that result.

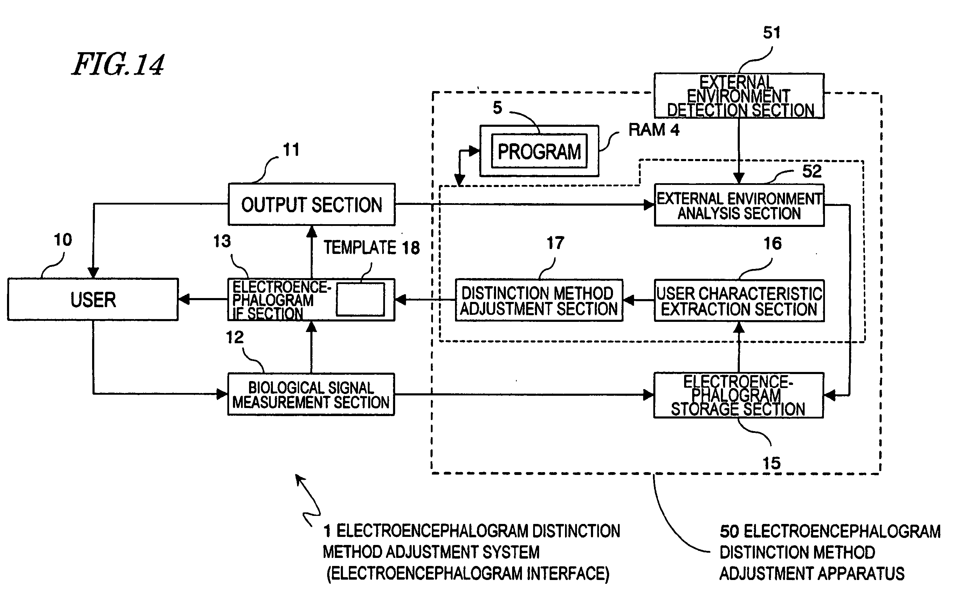

Hereinafter, with reference to FIG. **14****,** an electroencephalogram distinction method adjustment apparatus according to the present embodiment will be described.

FIG. **14** shows a functional block construction of the electroencephalogram interface system **1** according to the present embodiment. The electroencephalogram interface system **1** includes an electroencephalogram distinction method adjustment apparatus **50.**

The electroencephalogram distinction method adjustment apparatus **50** differs from the electroencephalogram distinction method adjustment apparatus **2** (FIG. **2**) in that the electroencephalogram distinction method adjustment apparatus **50** includes an external environment detection section **51** and an external environment analysis section **52,** instead of the content analysis section **14** of the electroencephalogram distinction method adjustment apparatus **2.** This means that the target of analysis is different between the electroencephalogram distinction method adjustment apparatus **50** and the electroencephalogram distinction method adjustment apparatus **2.**

Hereinafter, the electroencephalogram distinction method adjustment apparatus **50** will be described in detail. Among the component elements of the electroencephalogram distinction method adjustment apparatus **50,** those elements which are similar to those of the electroencephalogram distinction method adjustment apparatus **2** (FIG. **2**) will be denoted by like numerals, and the descriptions will be omitted.

The external environment detection section **51** detects a change in the environment within which the user **10** is situated. "An environment within which the user **10** is situated" means inside a room in the example shown in FIG. **1****.** Alternatively, when the user **10** has gone out of home with a wearable type device being worn, it means outdoor. When the user **10** is driving a car, it means the situation inside the car or around the road.

The external environment detection section **51** is a sensor which detects and outputs a state of the environment. More specifically, it is a microphone which acquires information of sounds in the environment within which the user **10** is situated and outputs the information, or a camera which acquires and outputs visual information around the user within the environment. However, these are examples. It is possible to use any sensor that is suitable for the environment to be detected.

Receiving an output signal from the external environment detection section **51** and, if necessary, an output from the output section **11** (presented content), the external environment analysis section **52** performs a signal analysis, and gives an instruction as the timing to store electroencephalograms in the electroencephalogram storage section **15.**

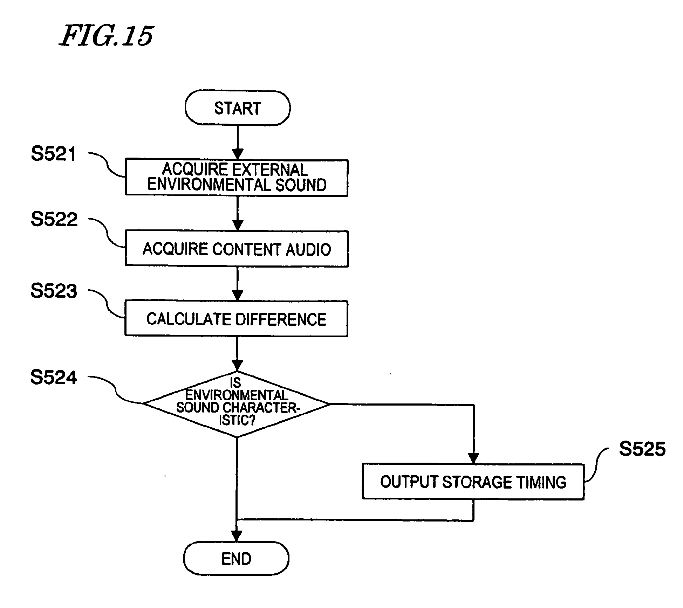

Next, with reference to FIG. **15****,** details of the analysis process for a change in an environment will be described. Hereinafter, as an example, it is envisaged that the user is viewing a content on television, and that an analysis process for a change in the environment is performed based on sounds in an indoor environment.

FIG. **15** shows a procedure of a process of analyzing a change in the environment concerning sounds in an indoor environment as shown in FIG. **1****.**

At step **S521,** the external environment detection section **51** acquires an external environmental sound. For example, when a microphone is installed near the electroencephalograph **12** worn by the user **10** or the like, external environmental sounds that are heard by the user **10** will be acquired by the microphone.

It is expected that voices and sounds from the television set **11** or the like will also be detected mixedly in the external environmental sounds that are acquired during viewing on the television set **11.** Therefore, the influences of the television set **11** are removed through the following steps.

At step **S522,** from within the output signal from the output section **11,** the external environment analysis section **52** acquires information concerning audio. As a result, the audio information which the content has is acquired.

At step **S523,** the external environment analysis section **52** removes the audio information of the content from the information of the external environmental sounds acquired by the external environment detection section **51.** As a result, a signal which derives only from the external environment can be extracted.

At step **S524,** the external environment analysis section **52** determines whether any characteristic sound is contained in the external environmental sound, by utilizing the signal which derives only from the external environment. Examples of characteristic sounds may be a "slam" of a closing door, a "ding dong" entry chime of an entryphone from a visiting guest, and the like. The physical quantities (waveform, audio level, etc.) of such sounds are **characterized in that** they rapidly rise in audio level, and that their audio levels affect the electroencephalograms as event-related potentials. So long as there are such characteristics of physical quantities of a sound, that sound can be considered as a sound which is characteristic of an external environmental sound. By acquiring an event-related potential of the electroencephalograms at this time, information for calibration can be obtained. Specifically, whether an amount of increase in sound level per unit time exceeds a predetermined threshold or not can be used as the determination criterion, for example. If it is determined as characteristic, control proceeds to step **S525;** if it is not determined as characteristic, the process is ended.

At step **S525,** since the change in sound is equal to or greater than the threshold, it is determined that an event-related potential to a sound stimulation is induced in the electroencephalograms of the user. Thus, a need to store data is determined, and this point in time is output as storage timing.

Through such processing, waveforms to be used for calibration can be obtained not only from the content information, but also from a characteristic change in the external environment.

In the above, an example has been illustrated of a calibration which is based on indoor environmental sounds during indoor viewing of television or the like. However, by detecting a change in a physical quantity in other external environments, a change in the environment can be detected and utilized for calibration.

For example, when one has gone out of home with a wearable electroencephalograph being worn, abruptly occurring outdoor sounds, e.g., a bell ringing at a station platform, storefront music, or in visual terms, flickering of a signboard or the like, may be detected as a change in a physical quantity, and a change in an event-related potential induced by such sound or flickering may be detected, whereby a change in the external environment can be detected. Similarly, in the case of driving a car, a honking of another car, flickering or switching of a traffic signal, or the like may be detected as a change in a physical quantity and utilized as a signal that may affect the electroencephalograms.

Thus, with the electroencephalogram distinction method adjustment apparatus of the present embodiment, information which is necessary for calibration is acquired also from changes in the environment of a situation within which the user is situated, without performing any explicit calibration. Therefore, an accurate determination of electroencephalograms is possible without requiring the user to spend time for calibration, thus providing an interface which is easy to manipulate.

### INDUSTRIAL APPLICABILITY

With an electroencephalogram distinction method adjustment apparatus according to the present invention and an electroencephalogram interface system incorporating such an apparatus, while a user is viewing a content, electroencephalograms (event-related potential) reflecting a characteristic pertaining to an individual difference of the user is extracted from within the content, and a determination method is adjusted. This is useful for improving the manipulability of any device whose determination method needs an improvement by allowing individual differences in electroencephalograms to be reflected thereupon, e.g., an information device or video/audio device incorporating a device manipulation interface which utilizes electroencephalograms. Other than interfaces utilizing electroencephalograms, the present apparatus also effectively functions for any device that needs correction for individual differences, e.g., a service providing device which detects a psychological state, emotional state, cognitive state or the like of a user and operates in accordance with such states.

The functions of such an apparatus can be realized by a computer program, for example, and therefore can be easily implemented without making significant modifications to a system.

## Claims

1. An adjustment apparatus used for adjusting an electroencephalogram distinction method in an electroencephalogram interface section (13) in an electroencephalogram interface system (1), the electroencephalogram interface system (1) including:
a biological signal measurement section (12) adapted to acquire an electroencephalogram signal from a user (10); and
the electroencephalogram interface section (13) which is adapted to distinguish a request of the user (10) based on the electroencephalogram signal and to identify a function which is in accordance with the request,
wherein the apparatus is adapted to be connected to each of the biological signal measurement section (12) and the electroencephalogram interface section (13), and
wherein the apparatus comprises:
an analysis section (14) adapted to measure and analyze a physical quantity corresponding to a visual and/or auditory stimulation given to the user (10), the physical quantity being measured and analyzed as a characteristic quantity of the stimulation, to detect a change in said characteristic quantity that affects an event-related potential contained in the electroencephalogram signal acquired by the biological signal measurement section (12), thus detecting a change in the stimulation,
wherein said visual and/or auditory stimulation is given from a content which the user (10) is viewing or from ambient light or environmental sound in an environment within which the user (10) exists;
a storage section (15) for storing the waveform of the event-related potential during a predetermined period at least spanning after a starting point which is a point in time when the change in the stimulation is detected;
a user characteristic extraction section (16) adapted to extract a characteristic quantity of the user (10) based on the stored waveform of the event-related potential; and
a distinction method adjustment section (17) adapted to, based on the extracted characteristic quantity of the user (10), adjust in the electroencephalogram interface section (13) the electroencephalogram distinction method for a request based on the electroencephalogram signal.

2. The adjustment apparatus of claim 1, wherein,
the stimulation is video and/or audio of the content presented to the user (10) by an output section (11), the adjustment apparatus being adapted to be connected to the output section (11); and
the analysis section (14) is adapted to analyze the data of the content to detect a change in the characteristic quantity of the stimulation that affects the event-related potential contained in the electroencephalogram signal.

3. The adjustment apparatus of claim 2, further comprising the output section (11) for, based on data of the content, presenting content to the user (10), wherein,
based on moving picture data, the output section (11) is adapted to display respectively a plurality of pictures which are switched one after another at a predetermined frequency, thus presenting a moving picture content; and
as a change in the characteristic quantity of the content, the analysis section (14) is adapted to detect a change in an image characteristic quantity of the consecutive plurality of images.

4. The adjustment apparatus of claim 3, wherein, as a change in the characteristic quantity of the content, the analysis section (14) is adapted to detect a change in at least one of luminance and hue.

5. The adjustment apparatus of claim 2, further comprising the output section (11) for, based on data of the content, presenting content to the user (10), wherein,
the output section (11) is adapted to present an audio content based on audio data; and
as a change in the characteristic quantity of the content, the analysis section (14) is adapted to detect a change in an output level of the audio.

6. The adjustment apparatus of claim 2, further comprising the output section (11) for, based on data of the content, presenting content to the user (10), wherein,
the output section (11) is adapted to present a moving picture content by displaying respectively a plurality of pictures which are switched one after another at a predetermined frequency, and also presents an audio content; and
as a change in the characteristic quantity of the content, the analysis section (14) detects a synchronized occurrence of a change in an image characteristic quantity of the consecutive plurality of images and a change in an output level of the audio.

7. The adjustment apparatus of claim 2, wherein,
the electroencephalogram interface section (13) is adapted to distinguish the request of the user (10) based on a threshold retained in advance and an amplitude of the event-related potential in the electroencephalogram signal;
the user characteristic extraction section is adapted to extract an amplitude of the stored event-related potential as the characteristic quantity of the user (10); and
based on the extracted characteristic quantity of the user (10), the distinction method adjustment section is adapted to adjust the threshold retained by the electroencephalogram interface section (13).

8. The adjustment apparatus of claim 2, wherein,
the electroencephalogram interface section (13) is adapted to distinguish the request of the user (10) based on a correlation between at least one waveform template retained in advance and the waveform of the event-related potential in the electroencephalogram signal;
the user characteristic extraction section is adapted to extract the stored waveform of the event-related potential as the characteristic quantity of the user (10); and
based on the extracted characteristic quantity of the user (10), the distinction method adjustment section is adapted to adjust the at least one waveform template retained by the electroencephalogram interface section (13).

9. The adjustment apparatus of claim 8, wherein,
based on the extracted characteristic quantity of the user (10), the distinction method adjustment section is adapted to change a value of the at least one waveform template and to set the at least one waveform template to the electroencephalogram interface section (13); and
the electroencephalogram interface section (13) is adapted to distinguish the request of the user (10) based on the at least one waveform template having been set.

10. The adjustment apparatus of claim 8, wherein,
the electroencephalogram interface section (13) is adapted to retain a plurality of waveform templates; and
the distinction method adjustment section is adapted to identify one of the plurality of waveform templates based on the extracted characteristic quantity of the user (10), and to instruct the electroencephalogram interface section (13) to set the one waveform template as a template for distinguishing the request of the user (10).

11. The adjustment apparatus of claim 2, wherein,
the electroencephalogram interface section (13) is adapted to distinguish the request of the user (10) based on a correlation between a waveform template retained in advance and the waveform of the event-related potential in the electroencephalogram signal;
the user characteristic extraction section is adapted to extract the stored waveform of the event-related potential as the characteristic quantity of the user (10); and
the distinction method adjustment section is adapted to instruct one of the electroencephalogram interface section (13) and the biological signal measurement section to adjust an amplitude of the electroencephalogram signal of the user (10) based on the extracted characteristic quantity of the user (10).

12. The adjustment apparatus of claim 2, wherein,
the user characteristic extraction section is adapted to output a signal when an amplitude of the waveform the event-related potential stored in the storage section is smaller than a predetermined threshold; and
based on the signal from the user characteristic extraction section, the distinction method adjustment section is adapted to instruct the electroencephalogram interface section (13) to output an alarm concerning acquisition of the electroencephalogram signal by the biological signal measurement section.

13. The adjustment apparatus of claim 1, wherein,
the stimulation is light and/or sound in an environment within which the user (10) exists; and
the analysis section (14) is adapted to detect light and/or sound in the environment, and to detect a change in the characteristic quantity of light and/or sound in the environment that affects the event-related potential contained in the electroencephalogram signal.

14. A method used for adjusting an electroencephalogram distinction method in an electroencephalogram interface section (13) in an electroencephalogram interface system (1), the electroencephalogram interface system (1) including:
a biological signal measurement section (12) adapted to acquire an electroencephalogram signal from a user (10); and
the electroencephalogram interface section (13) which is adapted to distinguish a request of the user (10) based on the electroencephalogram signal and to identify a function which is in accordance with the request,
wherein the method comprises the steps of:
measuring and analysing a physical quantity corresponding to a visual and/or auditory stimulation given to the user (10) from a content which the user (10) is viewing or from ambient light or environmental sound in an environment within which the user (10) exists, the physical quantity being measured as a characteristic quantity of the stimulation;
detecting a change in said characteristic quantity that affects an event-related potential contained in the electroencephalogram signal acquired by the biological signal measurement section (12), thus detecting a change in the stimulation;
storing a waveform of the event-related potential during a predetermined period at least spanning after a starting point which is a point in time when the change in the stimulation is detected;
extracting a characteristic quantity of the user (10) based on the stored waveform of the event-related potential; and
based on the extracted characteristic quantity of the user (10), adjusting in the electroencephalogram interface section (13) the electroencephalogram distinction method for a request based on the electroencephalogram signal.

15. A computer program to be executed by a computer implemented in an electroencephalogram distinction method adjustment apparatus (2) used for adjusting an electroencephalogram distinction method in an electroencephalogram interface section (13) in an electroencephalogram interface system (1), the electroencephalogram interface system (1) including:
a biological signal measurement section (12) adapted to acquire an electroencephalogram signal from a user (10); and
the electroencephalogram interface section (13) which is adapted to distinguish a request of the user (10) based on the electroencephalogram signal and to identify a function which is in accordance with the request,
wherein the apparatus (2) is connected to each of the biological signal measurement section (12) and the electroencephalogram interface section (13), and
wherein the computer program causes the computer to execute the steps of:
measuring a physical quantity corresponding to a visual and/or auditory stimulation given to the user (10) from a content which the user (10) is viewing or from ambient light or environmental sound in an environment within which the user (10) exists, the physical quantity being measured as a characteristic quantity of the stimulation;
detecting a change in said characteristic quantity that affects an event-related potential contained in the electroencephalogram signal acquired by the biological signal measurement section (12), thus detecting a change in the stimulation;
storing a waveform of the event-related potential during a predetermined period at least spanning after a starting point which is a point in time when the change in the stimulation is detected;
extracting a characteristic quantity of the user (10) based on the stored waveform of the event-related potential; and
based on the extracted characteristic quantity of the user (10), adjusting in the electroencephalogram interface section (13) the electroencephalogram distinction method for a request based on the electroencephalogram signal.

## Patentansprüche

1. Anpassungsvorrichtung, die dazu dient, ein Elektroenzephalogramm-Erkennungsverfahren in einem Elektroenzephalogramm-Schnittstellenabschnitt (13) in einem Elektroenzephalogramm-Schnittstellensystem (1) anzupassen, wobei das Elektroenzephalogramm-Schnittstellensystem (1) enthält:
einen Abschnitt (12) zum Messen eines biologischen Signals, der so eingerichtet ist, dass er ein Elektroenzephalogramm-Signal von einem Benutzer (10) erfasst; und
den Elektroenzephalogramm-Schnittstellenabschnitt (13), der so eingerichtet ist, dass er eine Anforderung des Benutzers (10) auf Basis des Elektroenzephalogramm-Signals erkennt und eine Funktion identifiziert, die der Anforderung entspricht,
wobei die Vorrichtung so eingerichtet ist, dass sie mit dem Abschnitt (12) zum Messen eines biologischen Signals und dem Elektroenzephalogramm-Schnittstellenabschnitt (13) verbunden ist, und
die Vorrichtung umfasst:
einen Analyseabschnitt (14), der so eingerichtet ist, dass er eine physikalische Größe misst und analysiert, die einer optischen und/oder akustischen Stimulation entspricht, die der Benutzer (10) erhält, wobei die physikalische Größe als eine charakteristische Größe der Stimulation gemessen und analysiert wird, um eine Änderung der charakteristischen Größe zu erfassen, die ein ereignisbezogenes Potential beeinflusst, das in dem durch den Abschnitt (12) zum Messen eines biologischen Signals erfassten Elektroenzephalogramm-Signal enthalten ist, um so eine Änderung in der Stimulation zu erfassen,
wobei die optische und/oder akustische Stimulation über einen Inhalt, den der Benutzer betrachtet, oder über ein Umgebungslicht oder einen Umgebungston in einer Umgebung, in der sich der Benutzer (10) befindet, erfolgt;
einen Speicherabschnitt (15) zum Speichern der Wellenform des ereignisbezogenen Potentials während eines vorgegebenen Zeitraums, der sich wenigstens ab einem Anfangspunkt, der ein Zeitpunkt ist, zu dem die Änderung in der Stimulation erfasst wird, erstreckt;
einen Abschnitt (16) zum Extrahieren einer Benutzer-Charakteristik, der so eingerichtet ist, dass er eine charakteristische Größe des Benutzers (10) auf Basis der gespeicherten Wellenform des ereignisbezogenen Potentials extrahiert; und
einen Abschnitt (17) zum Anpassen des Erkennungsverfahrens, der so eingerichtet ist, dass er auf Basis der extrahierten charakteristischen Größe des Benutzers (10) das Elektroenzephalogramm-Erkennungsverfahren in dem Elektroenzephalogramm-Schnittstellenabschnitt (13) für eine Anforderung auf Basis des Elektroenzephalogramm-Signals anpasst.

2. Anpassungsvorrichtung nach Anspruch 1, wobei
die Stimulation Bild und/oder Ton des Inhaltes ist, der dem Benutzer (10) über einen Ausgabeabschnitt (11) präsentiert wird, und die Anpassungsvorrichtung so eingerichtet ist, dass sie mit dem Ausgabeabschnitt (11) verbunden ist; und
der Analyseabschnitt (14) so eingerichtet ist, dass er die Daten des Inhaltes analysiert, um eine Änderung der charakteristischen Größe der Stimulation zu erfassen, die das in dem Elektroenzephalogramm-Signal enthaltene ereignisbezogene Potential beeinflusst.

3. Anpassungsvorrichtung nach Anspruch 2, die des Weiteren den Ausgabeabschnitt (11) umfasst, mit dem dem Benutzer (10) Inhalt auf Basis von Daten des Inhaltes präsentiert wird, wobei
der Ausgabeabschnitt (11) so eingerichtet ist, dass er auf Basis von Bewegtbild-Daten jeweils eine Vielzahl von Bildern anzeigt, die mit einer vorgegebenen Frequenz nacheinander gewechselt werden, um so einen Bewegtbild-Inhalt zu präsentieren; und
der Analyseabschnitt (14) so eingerichtet ist, dass er als eine Änderung der charakteristischen Größe des Inhalts eine Änderung einer charakteristischen Größe von Bildern der aufeinanderfolgenden Vielzahl von Bildern erfasst.

4. Anpassungsvorrichtung nach Anspruch 3, wobei der Analyseabschnitt (14) so eingerichtet ist, dass er als eine Änderung der charakteristischen Größe des Inhaltes eine Änderung von Helligkeit oder/und Farbton erfasst.

5. Anpassungsvorrichtung nach Anspruch 2, die des Weiteren den Ausgabeabschnitt (11) umfasst, mit dem dem Benutzer (10) Inhalt auf Basis von Daten des Inhaltes präsentiert wird, wobei
der Ausgabeabschnitt (11) so eingerichtet ist, dass er einen Ton-Inhalt auf Basis von Ton-Daten präsentiert; und
der Analyseabschnitt (14) so eingerichtet ist, dass er als eine Änderung der charakteristischen Größe des Inhaltes eine Änderung eines Ausgabepegels des Tons erfasst.

6. Anpassungsvorrichtung nach Anspruch 2, die des Weiteren den Ausgabeabschnitt (11) umfasst, mit dem dem Benutzer (10) Inhalt auf Basis von Daten des Inhaltes präsentiert wird, wobei
der Ausgabeabschnitt (11) so eingerichtet ist, dass er einen Bewegtbild-Inhalt präsentiert, indem er jeweils eine Vielzahl von Bildern anzeigt, die mit einer vorgegebenen Frequenz nacheinander gewechselt werden, und er des Weiteren einen Ton-Inhalt präsentiert; und der Analyseabschnitt (14) als eine Änderung der charakteristischen Größe des Inhaltes ein synchrones Auftreten einer Änderung einer charakteristischen Größe von Bildern der aufeinanderfolgenden Vielzahl von Bildern sowie eine Änderung eines Ausgangspegels des Tons erfasst.

7. Anpassungsvorrichtung nach Anspruch 2, wobei
der Elektroenzephalogramm-Schnittstellenabschnitt (13) so eingerichtet ist, dass er die Anforderung des Benutzers (10) auf Basis eines im Voraus gespeicherten Schwellenwertes und einer Amplitude des ereignisbezogenen Potentials in dem Elektroenzephalogramm-Signal erkennt;
der Abschnitt zum Extrahieren der Benutzer-Charakteristik so eingerichtet ist, dass er eine Amplitude des gespeicherten ereignisbezogenen Potentials als die charakteristische Größe des Benutzers (10) extrahiert; und
der Abschnitt zum Anpassen des Erkennungsverfahrens so eingerichtet ist, dass er den durch den Elektroenzephalogramm-Schnittstellenabschnitt (13) gespeicherten Schwellenwert auf Basis der extrahierten charakteristischen Größe des Benutzers (10) anpasst.

8. Anpassungsvorrichtung nach Anspruch 2, wobei
der Elektroenzephalogramm-Schnittstellenabschnitt (13) so eingerichtet ist, dass er die Anforderung des Benutzers (10) auf Basis einer Korrelation zwischen wenigstens einem Wellenform-Muster, das im voraus gespeichert wird, und der Wellenform des ereignisbezogenen Potentials in dem Elektroenzephalogramm-Signal erkennt;
der Abschnitt zum Extrahieren der Benutzer-Charakteristik so eingerichtet ist, dass er die gespeicherte Wellenform des ereignisbezogenen Potentials als die charakteristische Größe des Benutzers (10) extrahiert; und
der Abschnitt zum Anpassen des Erkennungsverfahrens so eingerichtet ist, dass er das wenigstens eine durch den Elektroenzephalogramm-Schnittstellenabschnitt (13) gespeicherte Wellenform-Muster auf Basis der extrahierten charakteristischen Größe des Benutzers (10) anpasst.

9. Anpassungsvorrichtung nach Anspruch 8, wobei
der Abschnitt zum Anpassen des Erkennungsverfahrens so eingerichtet ist, dass er auf Basis der extrahierten charakteristischen Größe des Benutzers (10) einen Wert des wenigstens einen Wellenform-Musters ändert und das wenigstens eine Wellenform-Muster für den Elektroenzephalogramm-Schnittstellenabschnitt (13) setzt; und
der Elektroenzephalogramm-Schnittstellenabschnitt (13) so eingerichtet ist, dass er die Anforderung des Benutzers (10) auf Basis des gesetzten wenigstens einen Wellenform-Musters erkennt.

10. Anpassungsvorrichtung nach Anspruch 8, wobei
der Elektroenzephalogramm-Schnittstellenabschnitt (13) so eingerichtet ist, dass er eine Vielzahl von Wellenform-Mustern speichert; und
der Abschnitt zum Anpassen des Erkennungsverfahrens so eingerichtet ist, dass er eines der Vielzahl von Wellenform-Mustern auf Basis der extrahierten charakteristischen Größe des Benutzers (10) identifiziert und den Elektroenzephalogramm-Schnittstellenabschnitt (13) anweist, das eine Wellenform-Muster als ein Muster zum Erkennen der Anforderung des Benutzers (10) zu setzen.

11. Anpassungsvorrichtung nach Anspruch 2, wobei
der Elektroenzephalogramm-Schnittstellenabschnitt (13) so eingerichtet ist, dass er die Anforderung des Benutzers (10) auf Basis einer Korrelation zwischen einem im voraus gespeicherten Wellenform-Muster und der Wellenform des ereignisbezogenen Potentials in dem Elektroenzephalogramm-Signal erkennt;
der Abschnitt zum Extrahieren der Benutzer-Charakteristik so eingerichtet ist, dass er die gespeicherte Wellenform des ereignisbezogenen Potentials als die charakteristische Grö-βe des Benutzers (10) extrahiert; und
der Abschnitt zum Anpassen des Erkennungsverfahrens so eingerichtet ist, dass er den Elektroenzephalogramm-Schnittstellenabschnitt (13) oder den Abschnitt zum Messen eines biologischen Signals anweist, eine Amplitude des Elektroenzephalogramm-Signals des Benutzers (10) auf Basis der extrahierten charakteristischen Größe des Benutzers (10) anzupassen.

12. Anpassungsvorrichtung nach Anspruch 2, wobei
der Abschnitt zum Extrahieren der Benutzer-Charakteristik so eingerichtet ist, dass er ein Signal ausgibt, wenn eine Amplitude der in dem Speicherabschnitt gespeicherten Wellenform des ereignisbezogenen Potentials unter einem vorgegebenen Schwellenwert liegt; und
der Abschnitt zum Anpassen des Erkennungsverfahrens so eingerichtet ist, dass er den Elektroenzephalogramm-Schnittstellenabschnitt (13) auf Basis des Signals von dem Abschnitt zum Extrahieren der Benutzer-Charakteristik anweist, eine Warnung bezüglich der Erfassung des Elektroenzephalogramm-Signals durch den Abschnitt zum Messen eines biologischen Signals auszugeben.

13. Anpassungsvorrichtung nach Anspruch 1, wobei
die Stimulation Licht und/oder Ton in einer Umgebung ist, in der sich der Benutzer (10) befindet; und
der Analyseabschnitt (14) so eingerichtet ist, dass er Licht und/oder Ton in der Umgebung erfasst und eine Änderung der charakteristischen Größe von Licht und/oder Ton in der Umgebung erfasst, die das in dem Elektroenzephalogramm-Signal enthaltene ereignisbezogene Potential beeinflusst.

14. Verfahren zum Anpassen eines Elektroenzephalogramm-Erkennungsverfahrens in einem Elektroenzephalogramm-Schnittstellenabschnitt (13) in einem Elektroenzephalogramm-Schnittstellensystem (1), wobei das Elektroenzephalogramm-Schnittstellensystem (1) enthält:
einen Abschnitt (12) zum Messen eines biologischen Signals, der so eingerichtet ist, dass er ein Elektroenzephalogramm-Signal von einem Benutzer (10) erfasst; und
den Elektroenzephalogramm-Schnittstellenabschnitt (13), der so eingerichtet ist, dass er eine Anforderung des Benutzers (10) auf Basis des Elektroenzephalogramm-Signals erkennt und eine Funktion identifiziert, die der Anforderung entspricht,
wobei das Verfahren die folgenden Schritte umfasst:
Messen und Analysieren einer physikalischen Größe, die einer optischen und/oder akustischen Stimulation entspricht, die der Benutzer (10) von einem Inhalt, den der Benutzer (10) betrachtet, oder von Umgebungslicht oder Umgebungston in einer Umgebung erhält, in der sich der Benutzer (10) befindet, wobei die physikalische Größe als eine charakteristische Größe der Stimulation gemessen wird;
Erfassen einer Änderung der charakteristischen Größe, die ein ereignisbezogenes Potential beeinflusst, das in dem durch den Abschnitt (12) zum Messen eines biologischen Signals erfassten Elektroenzephalogramm-Signal enthalten ist, um so eine Änderung in der Stimulation zu erfassen;
Speichern einer Wellenform des ereignisbezogenen Potentials während eines vorgegebenen Zeitraums, der sich wenigstens ab einem Anfangspunkt, der ein Zeitpunkt ist, zu dem die Änderung in der Stimulation erfasst wird, erstreckt;
Extrahieren einer charakteristischen Größe des Benutzers (10) auf Basis der gespeicherten Wellenform des ereignisbezogenen Potentials; und
Anpassen, in dem Elektroenzephalogramm-Schnittstellenabschnitt (13), des Elektroenzephalogramm-Erkennungsverfahrens für eine Anforderung auf Basis des Elektroenzephalogramm-Signals, wobei das Anpassen auf der extrahierten charakteristischen Größe des Benutzers (10) basiert.

15. Computerprogramm, das durch einen Computer ausgeführt wird, der in einer Vorrichtung (2) zum Anpassen eines Elektroenzephalogramm-Erkennungsverfahrens implementiert ist, das dazu dient, ein Elektroenzephalogramm-Erkennungsverfahren in einem Elektroenzephalogramm-Schnittstellenabschnitt (13) in einem Elektroenzephalogramm-Schnittstellensystem (1) anzupassen, wobei das Elektroenzephalogramm-Schnittstellensystem (1) enthält:
einen Abschnitt (12) zum Messen eines biologischen Signals, der so eingerichtet ist, dass er ein Elektroenzephalogramm-Signal von einem Benutzer (10) erfasst; und
den Elektroenzephalogramm-Schnittstellenabschnitt (13), der so eingerichtet ist, dass er eine Anforderung des Benutzers (10) auf Basis des Elektroenzephalogramm-Signals erkennt und eine Funktion identifiziert, die der Anforderung entspricht,
wobei die Vorrichtung (2) mit dem Abschnitt (12) zum Messen eines biologischen Signals und dem Elektroenzephalogramm-Schnittstellenabschnitt (13) verbunden ist; und
das Computerprogramm den Computer veranlasst, die folgenden Schritte auszuführen:
Messen und Analysieren einer physikalischen Größe, die einer optischen und/oder akustischen Stimulation entspricht, die der Benutzer (10) von einem Inhalt, den der Benutzer (10) betrachtet, oder von Umgebungslicht oder Umgebungston in einer Umgebung erhält, in der sich der Benutzer (10) befindet, wobei die physikalische Größe als eine charakteristische Größe der Stimulation gemessen wird;
Erfassen einer Änderung der charakteristischen Größe, die ein ereignisbezogenes Potential beeinflusst, das in dem durch den Abschnitt (12) zum Messen eines biologischen Signals erfassten Elektroenzephalogramm-Signal enthalten ist, um so eine Änderung in der Stimulation zu erfassen;
Speichern einer Wellenform des ereignisbezogenen Potentials während eines vorgegebenen Zeitraums, der sich wenigstens ab einem Anfangspunkt, der ein Zeitpunkt ist, zu dem die Änderung in der Stimulation erfasst wird, erstreckt;
Extrahieren einer charakteristischen Größe des Benutzers (10) auf Basis der gespeicherten Wellenform des ereignisbezogenen Potentials; und
Anpassen, in dem Elektroenzephalogramm-Schnittstellenabschnitt (13), des Elektroenzephalogramm-Erkennungsverfahrens für eine Anforderung auf Basis des Elektroenzephalogramm-Signals, wobei das Anpassen auf der extrahierten charakteristischen Größe des Benutzers (10) basiert.

## Revendications

1. Appareil d'ajustement utilisé pour ajuster un procédé de distinction d'électroencéphalogramme dans une section d'interface d'électroencéphalogramme (13) dans un système d'interface d'électroencéphalogramme (1), le système d'interface d'électroencéphalogramme (1) comprenant :
une section de mesure de signal biologique (12) adaptée pour acquérir un signal d'électroencéphalogramme d'un utilisateur (10) ; et
la section d'interface d'électroencéphalogramme (13), qui est adaptée pour distinguer une demande de l'utilisateur (10) en fonction du signal d'électroencéphalogramme et pour identifier une fonction conforme à la demande,
dans lequel l'appareil est adapté pour être connecté à chacune des sections de mesure de signal biologique (12) et d'interface d'électroencéphalogramme (13), et
dans lequel l'appareil comprend :
une section d'analyse (14) adaptée pour mesurer et analyser une quantité physique correspondant à une stimulation visuelle et/ou auditive procurée à l'utilisateur (10), la quantité physique étant mesurée et analysée sous forme d'une quantité caractéristique de la stimulation, pour détecter un changement dans ladite quantité caractéristique qui affecte un potentiel relatif à l'évènement contenu dans le signal d'électroencéphalogramme acquis par la section de mesure de signal biologique (12), de manière à détecter un changement dans la stimulation,
dans lequel ladite stimulation visuelle et/ou auditive est donnée à partir d'un contenu qui est visualisé par l'utilisateur (10) ou à partir de la lumière ambiante ou du son ambiant dans un environnement où se trouve l'utilisateur (10) ;
une section de stockage (15) pour stocker la forme d'onde du potentiel relatif à l'évènement durant une période prédéterminée qui s'étend au moins après un moment initial, qui est un moment où le changement dans la stimulation est détecté ;
une section d'extraction de caractéristique d'utilisateur (16) adaptée pour extraire une quantité caractéristique de l'utilisateur (10) en fonction de la forme d'onde stockée du potentiel relatif à l'évènement ; et
une section d'ajustement du procédé de distinction (17) adaptée pour, en fonction de la quantité caractéristique de l'utilisateur extraite (10), ajuster dans la section d'interface d'électroencéphalogramme (13) le procédé de distinction d'électroencéphalogramme pour une demande en fonction du signal d'électroencéphalogramme.

2. Appareil d'ajustement selon la revendication 1, dans lequel,
la stimulation est du vidéo et/ou de l'audio du contenu présenté à l'utilisateur (10) par une section de sortie (11), l'appareil d'ajustement étant adapté pour être connecté à la section de sortie (11) ; et
la section d'analyse (14) est adaptée pour analyser les données du contenu pour détecter un changement dans la quantité caractéristique de la stimulation qui affecte le potentiel relatif à l'évènement contenu dans le signal d'électroencéphalogramme.

3. Appareil d'ajustement selon la revendication 2, comprenant en outre la section de sortie (11) pour, en fonction de données du contenu, présenter du contenu à l'utilisateur (10), dans lequel,
en fonction de données d'image animée, la section de sortie (11) est adaptée pour afficher respectivement une pluralité d'images qui sont interchangées l'une après l'autre à une fréquence prédéterminée, présentant ainsi un contenu d'image animée ; et
comme changement de la quantité caractéristique du contenu, la section d'analyse (14) est adaptée pour détecter un changement d'une quantité caractéristique d'image de la pluralité consécutive d'images.

4. Appareil d'ajustement selon la revendication 3, dans lequel, comme changement de la quantité caractéristique du contenu, la section d'analyse (14) est adaptée pour détecter un changement d'au moins une caractéristique parmi la luminosité et la teinte.

5. Appareil d'ajustement selon la revendication 2, comprenant en outre la section de sortie (11) pour, en fonction de données du contenu, présenter du contenu à l'utilisateur (10), dans lequel,
la section de sortie (11) est adaptée pour présenter un contenu audio en fonction de données audio ; et
comme changement de la quantité caractéristique du contenu, la section d'analyse (14) est adaptée pour détecter un changement d'un niveau de sortie audio.

6. Appareil d'ajustement selon la revendication 2, comprenant en outre la section de sortie (11) pour, en fonction de données du contenu, présenter du contenu à l'utilisateur (10), dans lequel,
la section de sortie (11) est adaptée pour présenter un contenu à image animée en affichant respectivement une pluralité d'images qui sont interchangées l'une après l'autre à une fréquence prédéterminée, et pour présenter également un contenu audio ; et
comme changement de la quantité caractéristique du contenu, la section d'analyse (14) détecte une occurrence synchronisée d'un changement d'une quantité caractéristique d'image de la pluralité consécutive d'images et d'un changement d'un niveau de sortie de l'audio.

7. Appareil d'ajustement selon la revendication 2, dans lequel,
la section d'interface d'électroencéphalogramme (13) est adaptée pour distinguer la demande de l'utilisateur (10) en fonction d'un seuil retenu à l'avance et d'une amplitude du potentiel relatif à l'évènement dans le signal d'électroencéphalogramme ;
la section d'extraction de caractéristique d'utilisateur est adaptée pour extraire une amplitude du potentiel relatif à l'évènement stockée comme la quantité caractéristique de l'utilisateur (10) ; et
en fonction de la quantité caractéristique de l'utilisateur extraite (10), la section d'ajustement du procédé de distinction est adaptée pour ajuster le seuil retenu par la section d'interface d'électroencéphalogramme (13).

8. Appareil d'ajustement selon la revendication 2, dans lequel,
la section d'interface d'électroencéphalogramme (13) est adaptée pour distinguer la demande de l'utilisateur (10) en fonction d'une corrélation entre au moins un modèle de forme d'onde retenu à l'avance et la forme d'onde du potentiel relatif à l'évènement dans le signal d'électroencéphalogramme ;
la section d'extraction de caractéristique d'utilisateur est adaptée pour extraire la forme d'onde stockée du potentiel relatif à l'évènement comme quantité caractéristique de l'utilisateur (10) ; et
en fonction de la quantité caractéristique de l'utilisateur extraite (10), la section d'ajustement du procédé de distinction est adaptée pour ajuster l'au moins un modèle de forme d'onde retenu par la section d'interface d'électroencéphalogramme (13).

9. Appareil d'ajustement selon la revendication 8, dans lequel,
en fonction de la quantité caractéristique de l'utilisateur extraite (10), la section d'ajustement du procédé de distinction est adaptée pour changer une valeur de l'au moins un modèle de forme d'onde et pour fixer l'au moins un modèle de forme d'onde pour la section d'interface d'électroencéphalogramme (13) ; et
la section d'interface d'électroencéphalogramme (13) est adaptée pour distinguer la demande de l'utilisateur (10) en fonction de l'au moins un modèle de forme d'onde qui a été fixé.

10. Appareil d'ajustement selon la revendication 8, dans lequel,
la section d'interface d'électroencéphalogramme (13) est adaptée pour retenir une pluralité de modèles de forme d'onde ; et
la section d'ajustement du procédé de distinction est adaptée pour identifier un modèle de forme d'onde de la pluralité en fonction de la quantité caractéristique de l'utilisateur extraite (10), et pour commander à la section d'interface d'électroencéphalogramme (13) de fixer ledit modèle de forme d'onde comme modèle pour distinguer la demande de l'utilisateur (10).

11. Appareil d'ajustement selon la revendication 2, dans lequel,
la section d'interface d'électroencéphalogramme (13) est adaptée pour distinguer la demande de l'utilisateur (10) en fonction d'une corrélation entre un modèle de forme d'onde retenu à l'avance et la forme d'onde du potentiel relatif à l'évènement dans le signal d'électroencéphalogramme ;
la section d'extraction de caractéristique d'utilisateur est adaptée pour extraire la forme d'onde stockée du potentiel relatif à l'évènement comme quantité caractéristique de l'utilisateur (10) ; et
la section d'ajustement du procédé de distinction est adaptée pour commander soit la section d'interface d'électroencéphalogramme (13), soit la section de mesure de signal biologique pour ajuster une amplitude du signal d'électroencéphalogramme de l'utilisateur (10) en fonction de la quantité caractéristique de l'utilisateur extraite (10).

12. Appareil d'ajustement selon la revendication 2, dans lequel,
la section d'extraction de caractéristique d'utilisateur est adaptée pour sortir un signal lorsqu'une amplitude de la forme d'onde du potentiel relatif à l'évènement stockée dans la section de stockage est inférieure à un seuil prédéterminé ; et
en fonction du signal de la section d'extraction de caractéristique d'utilisateur, la section d'ajustement du procédé de distinction est adaptée pour commander la section d'interface d'électroencéphalogramme (13) pour qu'elle sorte une alarme concernant l'acquisition du signal d'électroencéphalogramme par la section de mesure de signal biologique.

13. Appareil d'ajustement selon la revendication 1, dans lequel,
la stimulation est une lumière et/ou un son dans un environnement où se trouve l'utilisateur (10) ; et
la section d'analyse (14) est adaptée pour détecter de la lumière et/ou du son dans l'environnement et pour détecter un changement dans la quantité caractéristique de lumière et/ou de son dans l'environnement qui affecte le potentiel relatif à l'évènement contenu dans le signal d'électroencéphalogramme.

14. Procédé utilisé pour ajuster un procédé de distinction d'électroencéphalogramme dans une section d'interface d'électroencéphalogramme (13) dans un système d'interface d'électroencéphalogramme (1), le système d'interface d'électroencéphalogramme (1) comprenant :
une section de mesure de signal biologique (12) adaptée pour acquérir un signal d'électroencéphalogramme d'un utilisateur (10) ; et
la section d'interface d'électroencéphalogramme (13) qui est adaptée pour distinguer une demande de l'utilisateur (10) en fonction du signal d'électroencéphalogramme et pour identifier une fonction qui est conforme à la demande,
dans lequel le procédé comprend les étapes consistant à :
mesurer et analyser une quantité physique correspondant à une stimulation visuelle et/ou auditive procurée à l'utilisateur (10) à partir d'un contenu qui est visualisé par l'utilisateur (10) ou à partir de la lumière ambiante ou du son ambiant dans un environnement où se trouve l'utilisateur (10), la quantité physique étant mesurée sous forme d'une quantité caractéristique de la stimulation ;
détecter un changement dans ladite quantité caractéristique qui affecte un potentiel relatif à l'évènement contenu dans le signal d'électroencéphalogramme acquis par la section de mesure de signal biologique (12), de manière à détecter un changement dans la stimulation ;
stocker une forme d'onde du potentiel relatif à l'évènement durant une période prédéterminée qui s'étend au moins après un moment initial, qui est un moment où le changement dans la stimulation est détecté ;
extraire une quantité caractéristique de l'utilisateur (10) en fonction de la forme d'onde stockée du potentiel relatif à l'évènement ; et
en fonction de la quantité caractéristique de l'utilisateur extraite (10), ajuster, dans la section d'interface d'électroencéphalogramme (13), le procédé de distinction d'électroencéphalogramme pour une demande en fonction du signal d'électroencéphalogramme.

15. Logiciel à exécuter par un ordinateur, mis en oeuvre dans un appareil d'ajustement du procédé de distinction d'électroencéphalogramme (2) utilisé pour ajuster un procédé de distinction d'électroencéphalogramme dans une section d'interface d'électroencéphalogramme (13) dans un système d'interface d'électroencéphalogramme (1), le système d'interface d'électroencéphalogramme (1) comprenant :
une section de mesure de signal biologique (12) adaptée pour acquérir un signal d'électroencéphalogramme d'un utilisateur (10) ; et
la section d'interface d'électroencéphalogramme (13) qui est adaptée pour distinguer une demande de l'utilisateur (10) en fonction du signal d'électroencéphalogramme et pour identifier une fonction qui est conforme à la demande,
dans lequel l'appareil (2) est connecté à chacune des sections de mesure de signal biologique (12) et d'interface d'électroencéphalogramme (13), et
dans lequel le logiciel commande à l'ordinateur de mettre en oeuvre les étapes consistant à :
mesurer une quantité physique correspondant à une stimulation visuelle et/ou auditive procurée à l'utilisateur (10) à partir d'un contenu qui est visualisé par l'utilisateur (10) ou à partir de la lumière ambiante ou du son ambiant dans un environnement où se trouve l'utilisateur (10), la quantité physique étant mesurée sous forme d'une quantité caractéristique de la stimulation,
détecter un changement dans ladite quantité caractéristique qui affecte un potentiel relatif à l'évènement contenu dans le signal d'électroencéphalogramme acquis par la section de mesure de signal biologique (12), de manière à détecter un changement dans la stimulation ;
stocker une forme d'onde du potentiel relatif à l'évènement durant une période prédéterminée qui s'étend au moins après un moment initial, qui est un moment où le changement dans la stimulation est détecté ;
extraire une quantité caractéristique de l'utilisateur (10) en fonction de la forme d'onde stockée du potentiel relatif à l'évènement ; et
en fonction de la quantité caractéristique de l'utilisateur extraite (10), ajuster, dans la section d'interface d'électroencéphalogramme (13), le procédé de distinction d'électroencéphalogramme pour une demande en fonction du signal d'électroencéphalogramme.
